# FASCICULE DE BREVET EUROPEEN

(11) **EP 0 766 696 B1**
(45) Date de publication et mention de la délivrance du brevet: **02.09.1998**
(21) Numéro de dépôt: 95923409.7
(22) Date de dépôt: 19.06.1995
(51) Int. Cl.: C07K 5/062, A61K 38/05

(54) **DERIVES DE THIAZOLIDINE, LEUR PREPARATION ET LES MEDICAMENTS LES CONTENANT**
THIAZOLIDINDERIVATE, IHRE HERSTELLUNG UND MEDIKAMENTE, DIE SIE ENTHALTEN
THIAZOLIDINE DERIVATIVES, THEIR PREPARATION AND DRUGS CONTAINING SAME

(30) Priorité: 22.06.1994 FR 9407627
(43) Date de publication de la demande: 09.04.1997
(73) Titulaire: RHONE-POULENC RORER S.A., 92160 Antony (FR)
(72) Inventeur: CAPET, Marc, F-91170 Vitry Chatillon (FR); DUBROEUCQ, Marie-Christine, F-95880 Enghein-les-Bains (FR); GUYON, Claude, F-94100 Saint-Maur-des-Fosses (FR); MANFRE, Franco, F-94450 Limeil-Brevannes (FR)
(74) Mandataire: Savina, Jacques
(86) Numéro de dépôt international: FR9500808
(87) Numéro de publication internationale: WO9535314

(56) Documents cités:
- EP-A- 0 527 069
- WO-A-94/15914
- WO-A-94/15915
- WO-A-94/15954
- WO-A-94/15955

## Description

La présente invention concerne des dérivés de thiazolidine de formule : 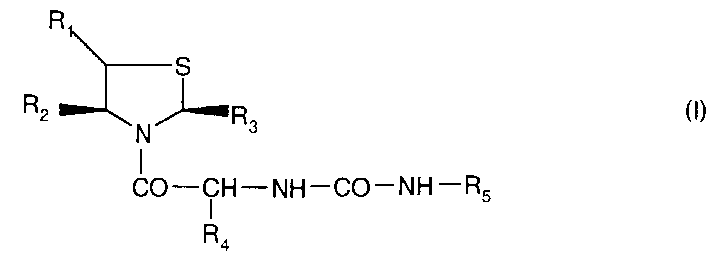 leurs sels, leur préparation et les médicaments les contenant.

Des dérivés de pyrrolidine et thiazolidine sont décrits comme antagonistes de la CCK dans le brevet EP527069.

Dans la formule (I),
R₁ représente un radical -(CH₂)ₙ-COORa ou -(CH₂)ₙ-CONRbRc,
R₂ représente un radical phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, trifluorométhyle et trifluorométhoxy,
R₃ représente un radical -COORd ou -CONReRf,
R₄ représente un atome d'hydrogène ou un radical alkyle,
R₅ représente un radical phényle éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-Rg, -SO₂-NH-SO₂-Rg, -CO-NH-CO-Rg, -CO-NH-SO₂-Rg, -B(OH)₂, -C(NH₂)=NOH, -SO₂-NH-Rh, -CO-NH-Rh, 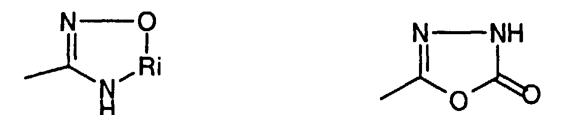 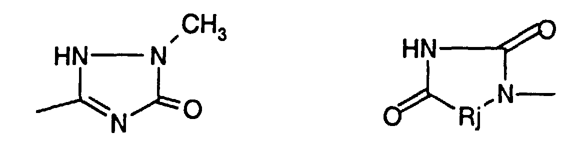 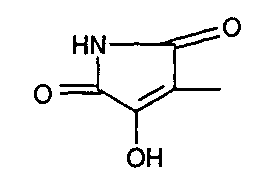 ou diméthyl-2,2 dioxo-4,6 dioxanne-1,3-yl-5,
Ra représente un atome d'hydrogène ou un radical alkyle,
Rb représente un atome d'hydrogène ou un radical alkyle,
Rc représente un radical alkyle, tétrazolyl-5, phénylalkyle dont le noyau phényle est éventuellement substitué par un ou plusieurs substituants choisis parmi les atomes d'halogène et les radicaux alkyle, alcoxy et alkylthio ou phényle éventuellement substitué par un ou plusieurs substituants choisis parmi les atomes d'halogène et les radicaux alkyle, alcoxy et alkylthio,
ou bien Rb et Rc forment avec l'atome d'azote auquel ils sont rattachés un hétérocycle mono ou polycyclique saturé ou insaturé contenant 4 à 9 atomes de carbone et un ou plusieurs hétéroatomes choisis parmi l'oxygène, l'azote ou le soufre et éventuellement substitué par un ou plusieurs radicaux alkyle,
Rd représente un radical alkyle,
Re représente un atome d'hydrogène ou un radical alkyle,
Rf représente un radical alkyle, cycloalkyle ou cycloalkylalkyle,
ou bien Re et Rf forment avec l'atome d'azote auquel ils sont rattachés un hétérocycle mono ou polycyclique saturé ou insaturé contenant 4 à 9 atomes de carbone et un ou plusieurs hétéroatomes choisis parmi l'oxygène, l'azote ou le soufre et éventuellement substitué par un ou plusieurs radicaux alkyle,
Rg représente un radical alkyle, cycloalkyle, trifluorométhyle, phényle éventuellement substitué par un ou plusieurs substituants choisis parmi les radicaux cyano, alcoxy, nitro, amino et les atomes d'halogène,
Rh représente un radical tétrazolyl-5,
Ri représente C=O ou S=O,
Rj représente O ou C=O,
n est égal à 0 ou 1
X représente un atome d'hydrogène ou un radical alkyle ou phénylalkyle,
alk représente un radical alkyle ou alkylène,
alk' représente un radical hydroxyalkyle, hydroxyalkylène, alcoxyalkyle ou alcoxyalkylène.

Dans les définitions qui précédent ou celles qui seront citées ci-après, sauf mention contraire, les radicaux et portions alkyle, alkylène et alcoxy contiennent 1 à 4 atomes de carbone en chaîne droite ou ramifiée et les radicaux ou portions cycloalkyle contiennent 3 à 6 atomes de carbone.

Les composés de formule (I) présentent des formes isomères. Ces isomères font également partie de l'invention.

Lorsque Rb et Rc ou bien Re et Rf forment avec l'atome d'azote auquel ils sont rattachés un hétérocycle mono ou polycyclique saturé ou insaturé contenant 4 à 9 atomes de carbone et un ou plusieurs hétéroatomes choisis parmi l'oxygène, l'azote ou le soufre et éventuellement substitué par un ou plusieurs radicaux alkyle celui-ci est de préférence un reste morpholinyle, thiomorpholinyle, pipéridyle éventuellement substitué par un ou plusieurs radicaux alkyle, pyrrolidinyle, tétrahydro-1,2,3,4 quinolyle ou N-alkyl pipérazinyle

Les composés de formule (I) pour lesquels R₁ représente un radical -(CH₂)ₙ-COORa, n est égal à 0 ou 1, Ra représente un radical alkyle et R₅ représente un radical phényle éventuellement substitué par un ou plusieurs substituants choisis parmi les atomes d'halogène et les radicaux alkyle, alcoxy, alkylthio, trifluorométhyle, alcoxycarbonyle, nitro, acyle, cyano, sulfamoyle, carbamoyle, alcoxyiminoalkyle, alcoxyaminocarbonyle, trifluorométhylsulfonamido, -alk-O-CO-alk, -alk-COOX, -alk-O-alk, -alk"COOX, -O-alk-COOX, -CH=CH-COOX, -CO-COOX, -alk-SO₃H (sous forme de sel), -CH=CH-alk', -S-alk-COOX, -SO-alk-COOX, -SO₂-alk-COOX, -O-CH₂-alk'-COOX, -CX=N-O-alk-COOX dans lesquels X est un radical alkyle ou phénylalkyle peuvent être préparés par action d'un dérivé de formule : 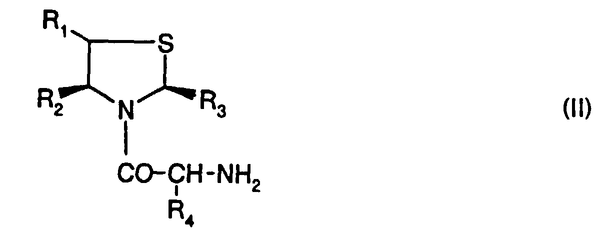 dans laquelle R₁ représente un radical -(CH₂)ₙ-COORa, n est égal à 0 ou 1, Ra représente un radical alkyle, R₂, R₃ et R₄ ont les mêmes significations que dans la formule (I), sur un phénylisocyanate dont le noyau phényle est éventuellement substitué par un ou plusieurs substituants choisis parmi les atomes d'halogène et les radicaux alkyle, alcoxy, alkylthio, trifluorométhyle, alcoxycarbonyle, nitro, acyle, cyano, sulfamoyle, carbamoyle, alcoxyiminoalkyle, alcoxyaminocarbonyle, trifluorométhylsulfonamido, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -S-alk-COOX, -SO-alk-COOX, -SO₂-alk-COOX, -O-CH₂-alk'-COOX, -CX=N-O-alk-COOX dans lesquels X est un radical alkyle ou phénylalkyle, alk représente un radical alkyle ou alkylène, alk' représente un radical hydroxyalkyle, hydroxyalkylène, alcoxyalkyle ou alcoxyalkylène.

Cette réaction s'effectue généralement au sein d'un solvant inerte tel que le tétrahydrofuranne, le diméthyllormamide, un solvant chloré (chloroforme, dichloro-1,2 éthane par exemple), un solvant aromatique (benzène, toluène par exemple), à une température comprise entre 10°C et la température d'ébullition du milieu réactionnel.

Les phénylisocyanates sont commercialisés ou peuvent être obtenus par application ou adaptation des méthodes décrites par R. RICHTER et coll., The Chemistry of Cyanate and their Thio Derivatives, S. PATAI, part 2, Wiley New York (1977) et dans les exemples.

Les dérivés de formule (II) peuvent être obtenus par action d'un dérivé de formule : 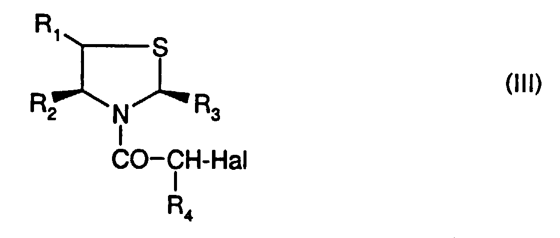 dans laquelle R₁ représente un radical -(CH₂)ₙ-COORa, n est égal à 0 ou 1, Ra représente un radical alkyle, R₂, R₃ et R₄ ont les mêmes significations que dans la formule (I) et Hal représente un atome d'halogène (chlore ou brome de préférence) sur l'azoture de sodium pour former l'azido correspondant sur lequel on fait réagir ensuite la triphénylphosphine puis de l'eau pour hydrolyser l'iminophosphorane.

La réaction avec l'azoture de sodium s'effectue généralement au sein d'un solvant inerte tel que le diméthylformamide, à une température voisine de 20°C. La réaction avec la triphénylphosphine s'effectue de préférence au sein d'un solvant inerte tel que le tétrahydrofuranne, à une température voisine de 20°C.

Les dérivés de formule (III) peuvent être obtenus par action d'un dérivé de formule : 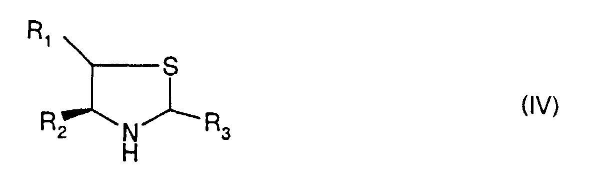 dans laquelle R₁ représente un radical -(CH₂)ₙ-COORa, n est égal à 0 ou 1, Ra représente un radical alkyle, R₂ et R₃ ont les mêmes significations que dans la formule (I), sur un dérivé de formule : 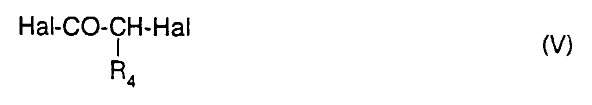 dans laquelle Hal représente un atome d'halogène (chlore ou brome de préférence) et R₄ a les mêmes significations que dans la formule (I).

Cette réaction s'effectue de préférence au sein d'un solvant aromatique tel que le toluène, en présence d'une base organique ou minérale telle qu'une trialkylamine (triéthylamine par exemple) ou l'hydrogénocarbonate de sodium, à la température d'ébullition du milieu réactionnel.

Les dérivés de formule (V) sont commercialisés ou peuvent être préparés à partir de l'acide correspondant que l'on transforme en chlorure d'acide par toute méthode connue de l'homme de l'art et notamment au moyen de chlorure d'oxalyle ou de chlorure de thionyle.

Les dérivés de formule (IV) pour lesquels R₁ représente un radical -(CH₂)ₙ-COORa, n est égal à 0, Ra représente un radical alkyle, R₃ représente un radical -COORd et Rd représente un radical alkyle peuvent être obtenus par action d'un dérivé de formule : 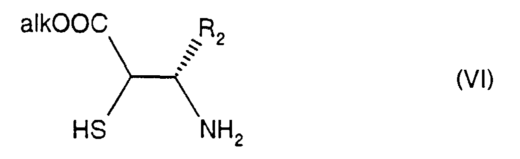 dans laquelle R₂ a les mêmes significations que dans la formule (I) et alk représente un radical alkyle, sur un dimorpholino-2,2 acétate d'alkyle.

Cette réaction s'effectue généralement au sein d'un solvant inerte tel qu'un alcool (méthanol par exemple), en présense d'une trialkylamine telle que la triéthylamine, à la température d'ébullition du milieu réactionnel.

Les dimorpholino-2,2 acétate d'alkyle peuvent être obtenus par application ou adaptation de la méthode décrite par R. HEYMES et coll., Bull. Soc. Chim., 2343-9 (1973).

Les dérivés de formule (VI) peuvent être obtenus par action de méthylate de sodium sur un dérivé de formule : 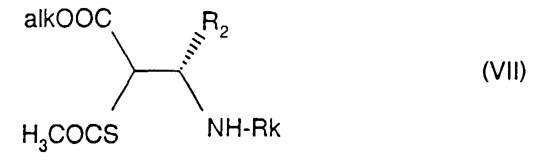 dans laquelle R₂ a les mêmes significations que dans la formule (I), alk représente un radical alkyle et Rk représente un groupe protecteur de la fonction amine tel que tert-butoxycarbonyle (Boc) puis déprotection de la fonction amine.

L'action du méthylate de sodium s'effectue de préférence au sein du méthanol, à une température voisine de 20°C et la déprotection s'effectue au moyen d'un acide minéral tel que l'acide chlorhydrique, à une température voisine de 20°C.

Les dérivés de formule (VII) peuvent être obtenus par action de thioacétate de potassium sur un dérivé de formule : 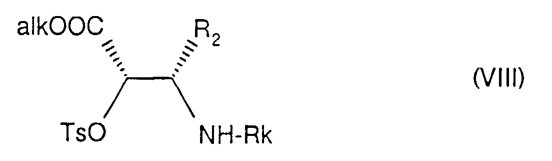 dans laquelle R₂ a les mêmes significations que dans la formule (I), Rk représente un groupe protecteur de la fonction amine tel que tert-butoxycarbonyle (Boc), alk représente un radical alkyle et Ts représente un reste tosyle.

Cette réaction s'effectue de préférence au sein d'un solvant inerte tel que l'acétone, à la température d'ébullition du milieu réactionnel.

Les dérivés de formule (VIII) peuvent être obtenus par tosylation des dérivés de formule : 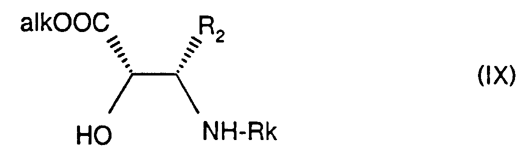 dans laquelle R₂ a les mêmes significations que dans la formule (I), Rk représente un groupe protecteur de la fonction amine tel que tert-butoxycarbonyle (Boc) et alk représente un radical alkyle.

Cette réaction s'effectue au moyen de chlorure de tosyle, au sein d'un solvant inerte tel que le dichlorométhane, en présence d'une trialkylamine telle que la triéthylamine, à une température voisine de 20°C ou au sein de la pyridine, à une température comprise entre 0 et 25°C.

Les dérivés de formule (IX) peuvent être préparés par application ou adaptation de la méthode décrite dans le brevet WO 93/17997.

Les dérivés de formule (IV) pour lesquels R₁ représente un radical -(CH₂)ₙ-COORa, n est égal à 1, Ra représente un radical alkyle, R₃ représente un radical -COORd et Rd représente un radical alkyle peuvent être obtenus par alcoolyse d'un dérivé de formule : 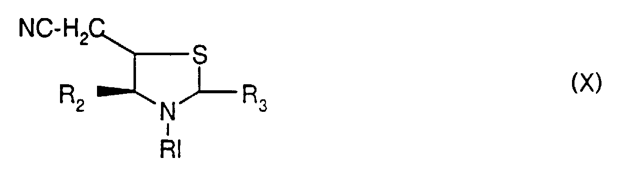 dans laquelle R₂ a les mêmes significations que dans la formule (I), R₃ représente un radical -COORd, Rd représente un radical alkyle et RI représente un groupe protecteur tel que benzyloxycarbonyle suivie d'une déprotection de la fonction amine.

Cette alcoolyse s'effectue généralement en milieu acide (acide chlorhydrique par exemple), au moyen d'un alcool (1-4C en chaîne droite ou ramifiée), à une température comprise entre 20°C et la température d'ébullition du milieu réactionnel. La déprotection s'effectue de préférence soit au moyen d'iodotriméthylsilane, au sein d'un solvant inerte tel qu'un solvant chloré (chloroforme par exemple), à une température comprise entre 20°C et la température d'ébullition du milieu réactionnel, soit par hydrogénolyse au moyen d'hydrogène, en présence d'un catalyseur d'hydrogénation tel que le charbon palladié, à une températute comprise entre 20°C et la température d'ébullition du milieu réactionnel, soit au moyen de formiate d'ammonium en présence de palladium, au sein d'un alcool tel que le méthanol, à la température d'ébullition du milieu réactionnel.

Les dérivés de formule (X) peuvent être obtenus par action d'un cyanure de métal alcalin sur un dérivé de formule : 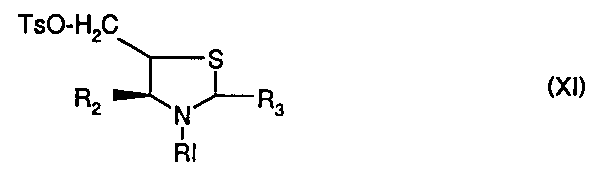 dans laquelle R₂ a les mêmes significations que dans la formule (I), R₃ représente un radical -COORd, Rd représente un radical alkyle, RI représente un groupe protecteur tel que benzyloxycarbonyle et Ts représente un reste tosyle.

Cette réaction s'effectue au sein d'un solvant inerte tel que le diméthylformamide, le tétrahydrofuranne ou un alcool (méthanol par exemple), à une température comprise entre 20°C et 100°C.

Les dérivés de formule (XI) peuvent être obtenus par tosylation du dérivé de formule : 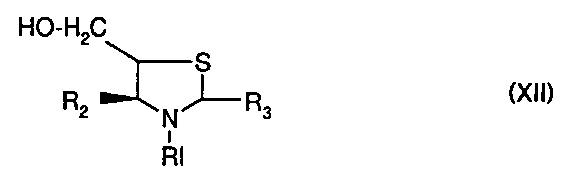 dans laquelle R₂ a les mêmes significations que dans la formule (I), R₃ représente un radical -COORd, Rd représente un radical alkyle et RI représente un groupe protecteur tel que benzyloxycarbonyle.

Cette réaction s'effectue généralement au moyen de chlorure de tosyle, au sein d'un solvant inerte tel que le dichlorométhane, en présence d'une trialkylamine telle que la triéthylamine, à une température voisine de 20°C ou au sein de la pyridine, à une température comprise entre 0 et 25°C.

Les dérivés de formule (XII) peuvent être obtenus par action de borohydrure de sodium sur un dérivé de formule : 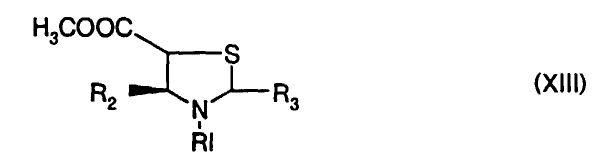 dans laquelle R₂ a les mêmes significations que dans la formule (I), R₃ représente un radical -COORd, Rd représente un radical alkyle et RI représente un groupe protecteur tel que benzyloxycarbonyle.

Cette réaction s'effectue au sein d'un alcool tel que le méthanol, à une température comprise entre 20°C et la température d'ébullition du milieu réactionnel.

Les dérivés de formule (XIII) peuvent être obtenus par protection de la fonction amine d'un dérivé de formule (IV) correspondant pour lequel R₁ représente un radical -(CH₂)ₙ-COORa, n est égal à 0, Ra représente un radical alkyle (1C), R₂ a les mêmes significations que dans la formule (I), R₃ représente un radical -COORd et Rd représente un radical alkyle au moyen d'un agent protecteur tel que le chloroformiate de benzyle.

Cette réaction s'effectue au sein d'un solvant inerte tel qu'un solvant chloré (dichlorométhane par exemple), en présence d'une trialkylamine (triéthylamine par exemple), à une température voisine de 20°C.

Les dérivés de formule (IV) pour lesquels R₁ représente un radical -(CH₂)ₙ-COORa, n est égal à 0 ou 1, Ra représente un radical alkyle, R₃ représente un radical -CONReRf , R₂, Re et Rf ont les mêmes significations que dans la formule (I) peuvent être obtenus par action d'un amine de formule HNReRf dans laquelle Re et Rf ont les mêmes significations que dans la formule (I), sur un acide de formule : 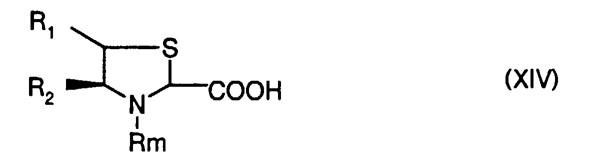 dans laquelle R₁ représente un radical -(CH₂)ₙ-COORa, n est égal à 0 ou 1, Ra représente un radical alkyle, R₂ a les mêmes significations que dans la formule (I) et Rm représente un radical méthoxycarbonyle ou (fluorényl-9 méthyl)-oxycarbonyle (Fmoc) puis déprotection de la fonction amine.

Cette réaction s'effectue en présence d'un agent de condensation peptidique tel qu'un carbodiimide (N,N'-dicyclohexylcarbodiimide par exemple) ou le N,N'-diimidazole carbonyle, au sein d'un solvant inerte tel qu'un éther (tétrahydrofuranne, dioxanne par exemple), un amide (diméthylformamide par exemple) ou un solvant chloré (chlorure de méthylène, chloroforme par exemple), à une température comprise entre 0°C et la température d'ébullition du milieu réactionnel. La déprotection de la fonction amine s'effectue par toute méthode connue qui ne touche pas les autres fonctions du produit; en particulier dans le cas où Rm représente un méthoxycarbonyle, on opère au moyen d'iodotriméthylsilane, au sein d'un solvant inerte tel que le chloroforme, à une température comprise entre 20°C et la température d'ébullition du milieu réactionnel et dans le cas où Rm représente un radical (fluorényl-9 méthyl)-oxycarbonyle, on opère au moyen d'une base telle que la pipéridine, la morpholine ou l'éthanolamine, au sein d'un solvant inerte tel que le diméthylformamide ou le dichlorométhane, à une température voisine de 20°C.

Les dérivés de formule (XIV) peuvent être obtenus par hydrolyse d'un dérivé de formule : 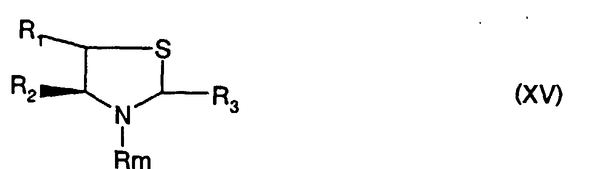 dans laquelle R₁ représente un radical -(CH₂)ₙ-COORa, n est égal à 0 ou 1, Ra représente un radical alkyle, R₂ a les mêmes significations que dans la formule (I), R₃ représente un radical tert-butoxycarbonyle et Rm représente un radical méthoxycarbonyle ou (fluorényl-9 méthyl)-oxycarbonyle (Fmoc).

Cette réaction s'effectue généralement au moyen d'iodotriméthylsilane, au sein d'un solvant inerte tel qu'un solvant chloré (chloroforme par exemple), à une température voisine de 25°C

Les dérivés de formule (XV) peuvent être obtenus par action d'un chlorure CI-Rm dans lequel Rm représente un radical méthoxycarbonyle ou (fluorényl-9 méthyl)-oxycarbonyle sur un dérivé de formule (IV) correspondant pour lequel R₁ représente un radical -(CH₂)ₙ-COORa, n est égal à 0 ou 1, Ra représente un radical alkyle, R₂ a les mêmes significations que dans la formule (I) et R₃ représente un radical tert-butoxycarbonyle.

Cette réaction s'effectue généralement au sein d'un solvant inerte tel qu'un solvant chloré (dichlorométhane par exemple), en présence d'une trialkylamine (triéthylamine par exemple), à une température voisine de 25°C.

Les composés de formule (I) pour lesquels R₁ représente un radical -(CH₂)ₙ-COORa, n est égal à 0 ou 1 et Ra est un radical alkyle peuvent également être préparés par action d'un dérivé réactif de l'acide carbamique, obtenu éventuellement in situ par action d'un dérivé réactif de l'acide carbonique choisi parmi le N,N'-diimidazole carbonyle, le phosgène, le diphosgène, le triphosgène et le chloroformiate de p-nitrophényle sur un dérivé de formule (II) pour lequel R₁ représente un radical -(CH₂)n-COORa, Ra représente un radical alkyle, R₂, R₃ et R₄ ont les mêmes significations que dans la formule (I) sur une aniline dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-Rg, -SO₂-NH-SO₂-Rg, -CO-NH-CO-Rg, -CO-NH-SO₂-Rg, -B(OH)₂, -C(NH₂)=NOH, -SO₂-NH-Rₕ, -CO-NH-Rₕ, 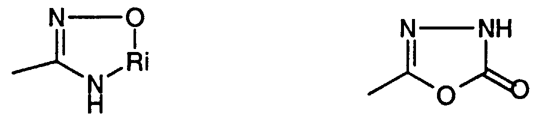 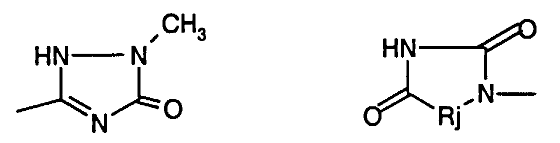 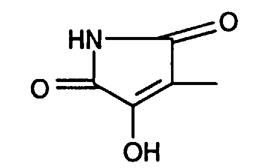 ou diméthyl-2,2 dioxo-4,6 dioxanne-1,3-yl-5, Ri représente C=O ou S=O, Rj représente O ou C=O, n est égal à 0 ou 1, X représente un atome d'hydrogène ou un radical alkyle ou phénylalkyle, alk représente un radical alkyle ou alkylène et alk' représente un radical hydroxyalkyle, hydroxyalkylène, alcoxyalkyle ou alcoxyalkylène.

Cette réaction s'effectue généralement au sein d'un solvant inerte tel que le tétrahydrofuranne, le diméthylformamide, un solvant chloré (chloroforme, dichloro-1,2 éthane par exemple) ou un solvant aromatique (benzène, toluène par exemple), à une température comprise entre 20°C et la température d'ébullition du solvant.

Le dérivé réactif de l'acide carbamique peut être obtenu dans les mêmes conditions de solvant et de température.

Les anilines éventuellement substituées sont commercialisées ou peuvent être obtenues par application ou adaptation des méthodes décrites par R. SCHRÖTER, Methoden der organischen Chemie, Houben Weil, Band XI/1, p 360; G.J. ESSELEN et coll., J. Am. Chem. Soc., 36, 322 (1914); G. ADRIANT et coll., Bull. Soc. Chim. Fr, 1511 (1970); W.A. JACOBS et coll., J. Am. Chem. Soc., 39, 2438 (1917) et J. Am. Chem. Soc., 39, 1438 (1917).

Les composés de formule (I) pour lesquels R₁ représente un radical -(CH₂)ₙ-CONRbRc peuvent être préparés par action d'un composé de formule (I) correspondant pour lequel R₁ représente un radical -(CH₂)ₙ-COORa, n est égal à 0 ou 1 et Ra représente un atome d'hydrogène, sur une amine de formule HNRbRc dans laquelle Rb et Rc ont les mêmes significations que dans la formule (I).

Cette réaction s'effectue généralement en présence d'un agent de condensation utilisé en chimie peptidique tel qu'un carbodiimide (par exemple le N,N'-dicyclohexylcarbodiimide) ou le N,N'-diimidazole carbonyle, dans un solvant inerte tel qu'un éther (tétrahydrofuranne, dioxanne par exemple), un amide (N,N-diméthyl-formamide) ou un solvant chloré (chlorure de méthylène, dichloro-1,2 éthane, chloroforme par exemple) à une température comprise entre 0°C et la température de reflux du mélange réactionnel.

Les composés de formule (I) pour lesquels R₁ représente un radical -(CH₂)ₙ-COORa, n est égal à 0 ou 1 et Ra représente un atome d'hydrogène peuvent être préparés par hydrolyse des composés de formule (I) correspondants pour lesquels R₁ représente un radical -(CH₂)ₙ-COORa pour lequel n est égal à 0 ou 1 et Ra représente un radical alkyle.

Cette réaction s'effectue généralement au sein d'un solvant inerte tel que l'eau, le tétrahydrofuranne ou un mélange de ces solvants, en présence d'un hydroxyde de métal alcalin tel que la soude, à une température comprise entre 0 et 25°C.

Les composés de formule (I) pour lesquels R₅ représente un radical phényle substitué par un radical comportant un reste COOX dans lequel X représente un atome d'hydrogène peuvent également être préparés par hydrogénolyse des esters benzyliques correspondants.

Cette réaction s'effectue généralement au sein d'un solvant inerte tel qu'un alcool (méthanol par exemple), au moyen d'hydrogène ou de formiate d'ammonium, en présence d'un catalyseur d'hydrogénation tel que le charbon palladié, à une température comprise entre 20°C et la température d'ébullition du milieu réactionnel.

Il est entendu pour l'homme de métier que, pour la mise en oeuvre des procédés selon l'invention décrits précédemment, il peut être nécessaire afin d'éviter des réactions secondaires d'introduire des groupes protecteurs des fonctions amine, alcool, acide, cétone tels que ceux décrits par T. W. GREENE, "protective groups in organic synthesis", John Wiley and Sons, New York. Par exemple les fonctions amine peuvent être bloquées sous forme de carbamates de tert-butyle ou de méthyle puis régénérées au moyen d'iodotriméthylsilane, de carbamate de fluorényl-9 méthyle puis régénérées au moyen d'une base telle que la pipéridine ou la morpholine ou de carbamates de benzyle puis régénérées par hydrogénation après avoir mis en oeuvre le procédé selon l'invention. Les fonctions alcool peuvent par exemple être bloquées sous forme de benzoate puis régénérées par hydrolyse en milieu alcalin après avoir mis en oeuvre le procédé selon l'invention. Les fonctions cétone peuvent être bloquées sous forme de dioxolanne-1,3 puis régénérées au moyen de d'un mélange acide chlorhydrique-acide acétique.

Les énantiomères des composés de formule (I) peuvent être obtenus par dédoublement des racémiques par exemple par chromatographie sur colonne chirale ou par synthèse à partir des précurseurs chiraux.

Les composés de formule (I) peuvent être purifiés par les méthodes connues habituelles, par exemple par cristallisation, chromatographie ou extractions.

Les composés de formule (I) comportant un reste basique peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) comportant un reste acide peuvent éventuellement être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac, d'une amine ou d'un sel d'une amine sur un composé de formule (I), dans un solvant. Le sel formé est séparé par les méthodes habituelles.

Ces sels font également partie de l'invention.

Comme exemples de sels pharmaceutiquement acceptables, peuvent être cités les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalinoterreux (calcium, magnésium), le sel d'ammonium, les sels de bases azotées (éthanolamine, triméthylamine, méthylamine, benzylamine, N-benzyl-β-phénéthylamine, choline, arginine, leucine, lysine, N-méthyl glucamine).

Les composés de formule (I) présentent des propriétés pharmacologiques intéressantes. Ces composés possèdent une forte affinité pour les récepteurs de la cholécystokinine (CCK) et de la gastrine et sont donc utiles dans le traitement et la prévention des désordres liés à la CCK et à la gastrine au niveau du système nerveux et de l'appareil gastrointestinal.

C'est ainsi que ces composés peuvent être utilisés pour le traitement ou la prévention des psychoses, des troubles anxieux, de la dépression, de la neurodégénération, des attaques de panique, de la maladie de Parkinson, de la diskynésie tardive, du syndrôme du colon irritable, de la pancréatite aiguë, des ulcères, des désordres de la motilité intestinale, de certaines tumeurs sensibles à la CCK, comme régulateur de l'appétit, dans le sevrage aux traitements chroniques et abus d'alcool ou de médicaments et comme constricteur de la pupille de l'oeil.

Ces composés ont également un effet de potentialisation sur l'activité analgésique des médicaments narcotiques et non narcotiques. En outre, ils peuvent avoir un effet analgésique propre.

Par ailleurs, les composés ayant une forte affinité pour les récepteurs CCK modifient les capacités de mémorisation. En conséquence, ces composés peuvent être efficaces dans les troubles de la mémoire.

L'affinité des composés de formule (I) pour les récepteurs CCK a été déterminée selon une technique inspirée de celle de A. SAITO et coll . (J. Neuro. Chem., 37, 483-490 (1981)) au niveau du cortex cérébral et au niveau du pancréas.

Dans ces tests, la Cl₅₀ des composés de formule (I) est généralement inférieure ou égale à 1000 nM.

Par ailleurs, il est connu que les produits qui reconnaissent les récepteurs centraux de la CCK ont une spécificité similaire pour les récepteurs de la gastrine dans le tractus gastrointestinal (BOCK et coll., J. Med. Chem., 32, 16-23 (1989); REYFELD et coll., Am. J. Physiol., 240, G255-266 (1981); BEINFELD et coll., Neuropeptides, 3, 411-427 (1983).

Les composés de formule (I) présentent une toxicité faible. Leur DL₅₀ est généralement supérieure à 40 mg/kg par voie sous cutanée chez la souris.

D'un intérêt particulier sont les composés de formule (I) pour lesquels
R₁ représente un radical -(CH₂)ₙ-COORa ou -(CH₂)ₙ-CONRbRc,
R₂ représente un radical phényle éventuellement substitué par un ou plusieurs substituants choisis parmi les atomes d'halogène et les radicaux alkyle, alcoxy, hydroxy, alcoxycarbonyle, trifluorométhyle et trifluorométhoxy,
R₃ représente un radical -COORd ou -CONReRf,
R₄ représente un atome d'hydrogène ou un radical alkyle (1 ou 2C),
R₅ représente un radical phényle éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H,
Ra représente un atome d'hydrogène ou un radical alkyle,
Rb représente un atome d'hydrogène ou un radical alkyle,
Rc représente un radical alkyle, tétrazolyl-5, phénylalkyle dont le noyau phényle est éventuellement substitué par un ou plusieurs substituants choisis parmi les atomes d'halogène et les radicaux alkyle, alcoxy et alkylthio ou phényle éventuellement substitué par un ou plusieurs substituants choisis parmi les atomes d'halogène et les radicaux alkyle, alcoxy et alkylthio,
ou bien Rb et Rc forment avec l'atome d'azote auquel ils sont rattachés un hétérocycle mono ou polycyclique saturé ou insaturé contenant 4 à 9 atomes de carbone et un ou plusieurs hétéroatomes choisis parmi l'oxygène, l'azote ou le soufre et éventuellement substitué par un ou plusieurs radicaux alkyle,
Rd représente un radical alkyle,
Re représente un atome d'hydrogène ou un radical alkyle,
Rf représente un radical alkyle, cycloalkyle ou cycloalkylalkyle,
ou bien Re et Rf forment avec l'atome d'azote auquel ils sont rattachés un hétérocycle mono ou polycyclique saturé ou insaturé contenant 4 à 9 atomes de carbone et un ou plusieurs hétéroatomes choisis parmi l'oxygène, l'azote ou le soufre et éventuellement substitué par un ou plusieurs radicaux alkyle,
n est égal à 0 ou 1
X représente un atome d'hydrogène ou un radical alkyle ou phénylalkyle,
alk représente un radical alkyle ou alkylène,
alk' représente un radical hydroxyalkyle, hydroxyalkylène, alcoxyalkyle ou alcoxyalkylène, leurs isomères et leurs sels.

Parmi ces composés les préférés sont les suivants :
méthoxycarbonyl-5 {[(méthyl-3 phényl)-3 uréido]-2 acétyl}-3 phényl-4 thiazolidinecarboxylate-2 de tert-butyle-(2S,4S,5R),
acide {[(tert-butoxycarbonyl-2 carboxy-5 phényl-4 thiazolidinyl-3-(2S,4S,5R))-2 oxo-2 éthyl]-3 uréido}-3 phénylacétique,
acide {{[(tert-butoxycarbonyl-2 carboxy-5 phényl-4 thiazolidinyl-3-(2S, 4S, 5R))-2 oxo-2 éthyl]-3 uréido}-3 phényl}-2 propionique-(S),
méthoxycarbonyl-5 {[(méthyl-3 phényl)-3 uréido]-2 acétyl}-3 (fluoro-2 phényl)-4 thiazolidinecarboxylate-2 de tert-butyle-(2S,4S,5R),
acide tert-butoxycarbonyl-2 {[(méthyl-3 phényl)-3 uréido]-2 acétyl}-3 (fluoro-2 phényl)-4 thiazolidinecarboxylique-5-(2S,4S,5R),
et leurs sels.

Les exemples suivants illustrent l'invention.

### EXEMPLE 1

Dans un ballon contenant 0,14 g d'(amino-2 acétyl)-3 méthoxycarbonyl-5 phényl-4 thiazolidinecarboxylate-2 de tert-butyle-(2S,4S,5R) en solution dans 10 ml de tétrahydrofuranne on ajoute 50 µl d'isocyanate de méthyl-3 phényle. Le mélange réactionnel est agité pendant 12 heures à une temperature voisine de 25°C puis concentré à sec sous pression réduite à 35°C. Le produit brut est purifié par chromatographie sur silice [éluant : acétate d'éthyle-cyclohexane (40/60 en volumes)]. Les fractions contenant le produit attendu sont réunies et concentrées à sec sous pression réduite à 40°C. On obtient ainsi, 0,13 g de méthoxycarbonyl-5 ([(méthyl-3 phényl)-3 uréido]-2 acétyl}-3 phényl-4 thiazolidinecarboxylate-2 de tert-butyle-(2S,4S,5R) sous forme d'une meringue blanche, [α]_{D}²⁵ = -9,5° ± 0,8° (c = 1,0; CH₃OH), R.M.N. ¹H : (200 MHz, (CD₃)₂SO avec ajout de quelques gouttes de CD₃COOD, à une température de 373 K, δ en ppm) : 1,51 (s, 9H : CH₃ du tert-butyle); 2,28 (s, 3H : ArCH₃); 3,69 et 4,05 (respectivement d large et d, J= 17 Hz, 1H chacun : COCH₂N); 3,78 (s, 3H : COOCH₃); 4,31 (d, J = 3,5 Hz, 1H : en C5 du cycle); 5,75 (s, 1H : H en C2 du cycle); 5,86 (d, J = 3,5 Hz, 1H : H en C4 du cycle); 6,76 (d large, J = 7,5 Hz, 1H : H aromatique (H en ortho du CH₃)); 7,10 (t, J = 7,5 Hz, 1H : H aromatique (H en méta du CH₃)); de 7,10 à 7,50 (mt, 5H : H aromatiques); 7,73 (mt, 2H : H ortho du phényle).
A. L'(amino-2 acétyl)-3 méthoxycarbonyl-5 phényl-4 thiazolidinecarboxylate-2 de tert-butyle-(2S,4S,5R) peut être préparé de la manière suivante : à une solution de 0,42 g d'(azido-2 acétyl)-3 méthoxycarbonyl-5 phényl-4 thiazolidinecarboxylate-2 de tert-butyle-(2S,4S,5R) dans 10 ml de tétrahydrofuranne, on ajoute à une température voisine de 25°C, 0,27g de triphénylphosphine. Le mélange réactionnel est agité pendant 15 minutes à une température voisine de 25°C puis on ajoute 20 µl d'eau, poursuit l'agitation pendant 12 heures à une température voisine de 25°C et concentre à sec sous pression réduite à 35°C. Le résidu obtenu est dilué par 20 ml d'oxyde de diéthyle et extrait par 10 ml d'une solution aqueuse 0,1N d'acide chlorhydrique. La phase aqueuse est séparée par décantation, amenée à pH 9 par addition d'une solution aqueuse 1N d'hydroxyde de sodium et extraite par 3 fois 20 ml d'oxyde de diéthyle. Les phases organiques réunies sont lavées par 20 ml d'eau, séchées sur sulfate de magnésium et concentrées à sec sous pression réduite à 40°C. On obtient ainsi 0,14 g d'(amino-2 acétyl)-3 méthoxycarbonyl-5 phényl-4 thiazolidinecarboxylate-2 de tert-butyle-(4S) mélange des isomères en position 2 et 5 sous forme d'une huile jaune utilisée telle quelle dans les synthèses ultérieures.
B. L'(azido-2 acétyl)-3 méthoxycarbonyl-5 phényl-4 thiazolidinecarboxylate-2 de tert-butyle-(2S,4S,5R) peut être préparé de la manière suivante : à une solution de 0,46 g de (bromo-2 acétyl)-3 méthoxycarbonyl-5 phényl-4 thiazolidinecarboxylate-2 de tert-butyle-(2S,4S,5R) dans 5 ml de diméthylformamide, on ajoute, à une température voisine de 20°C, une solution de 0,07 g d'azoture de sodium dans 5 ml de diméthylformamide. Le milieu réactionnel est agité pendant 1 heure à cette température, puis hydrolysé par addition de 15 ml d'eau et extrait par 2 fois 20 ml d'oxyde de diéthyle. Les phases organiques réunies sont lavées par 2 fois 20 ml d'eau, séchées sur sulfate de magnésium et concentrées à sec sous pression réduite à 25°C. On obtient ainsi 0,5 g d'(azido-2 acétyl)-3 méthoxycarbonyl-5 phényl-4 thiazolidinecarboxylate-2 de tert-butyle-(2S,4S,5R) sous forme d'une huile jaune utilisée telle quelle dans les synthèses ultérieures.
C. Le (bromo-2 acétyl)-3 méthoxycarbonyl-5 phényl-4 thiazolidinecarboxylate-2 de tert-butyle-(2S,4S,5R) peut être préparé de la manière suivante : à un mélange contenant de 0,86 g de méthoxycarbonyl-5 phényl-4 thiazolidinecarboxylate-2 de tert-butyle-(4S) mélange des isomères en positions 2 et 5 et 0,22 g d'hydrogénocarbonate de sodium dans 15 ml de toluène on ajoute une solution de 0,54 g de bromure de bromoacétyle dans 5 ml de toluène. Le milieu réactionnel est chauffé à reflux pendant 1,5 heure, puis après retour à une température voisine de 20°C, dilué par 40 ml d'acétate d'éthyle, lavé successivement par 20 ml d'une solution aqueuse saturée d'hydrogénocarbonate de sodium et par 20 ml d'eau, séché sur sulfate de magnésium et concentré à sec sous pression réduite à 40°C. Le produit brut est purifié par chromatographie sur silice [éluant : cyclohexane-acétate d'éthyle (90/10 puis 80/20 en volumes)]. Les fractions contenant le produit sont réunies et concentrées à sec sous pression réduite à 30°C. On obtient ainsi 0,19 g de (bromo-2 acétyl)-3 méthoxycarbonyl-5 phényl-4 thiazolidinecarboxylate-2 de tert-butyle-(2S,4S,5R) (élué le premier) sous forme d'une huile jaune utilisée telle quelle dans les synthèses ultérieures et 0,23 g de (bromo-2 acétyl)-3 méthoxycarbonyl-5 phényl-4 thiazolidinecarboxylate-2 de tert-butyle-(2S,4S,5S) qui après battage dans l'oxyde de diisopropyle, se présente sous la forme d'un solide crème fondant à 137°C.
D. Le méthoxycarbonyl-5 phényl-4 thiazolidinecarboxylate-2 de tert-butyle(48) mélange des isomères en positions 2 et 5 peut être préparé de la manière suivante : à une solution de 18 g de chlorhydrate d'amino-3 mercapto-2 phényl-3 propionate de méthyle-(2RS,3S) dans 400 ml de méthanol on ajoute sous atmosphère d'argon, 15,3 ml de triéthylamine et 34,4 g de dimorpholino-2,2 acétate de tert-butyle. Le milieu réactionnel est chauffé au reflux pendant 2,5 heures puis après retour à une température voisine de 20°C, évaporé à sec sous pression réduite. Le résidu huileux obtenu est dilué par 50 ml d'acétate d'éthyle, lavé successivement par 2 fois 50 ml d'eau et 50 ml d'une solution saturée de chlorure de sodium, séché sur sulfate de magnésium et concentré à sec sous pression réduite à une température voisine de 40°C. Le produit brut est purifié par chromatographie sur silice [éluant : éther de pétrole-oxyde de diisopropyle (80/20 en volumes)]. On obtient ainsi 8 g de méthoxycarbonyl-5 phényl-4 thiazolidinecarboxylate-2 de tert-butyle-(4S) mélange des isomères en positions 2 et 5 sous forme d'une huile jaune utilisée telle quelle dans les synthèses ultérieures.
E. Le chlorhydrate d'amino-3 mercapto-2 phényl-3 propionate de méthyle-(2RS,3S) peut être préparé de la manière suivante : dans un ballon purgé à l'argon et à l'abri de la lumière on introduit une solution de 13,5 g de tert-butoxycarbonylamino-3 mercapto-2 phényl-3 propionate de méthyle-(2RS, 3S) dans 150 ml de méthanol. A ce mélange refroidi à une température voisine de 5°C on ajoute lentement 45 ml d'une solution méthanolique 8N d'acide chlorhydrique. L'agitation est poursuivie pendant 3 heures à une température voisine de 20°C, puis le milieu réactionnel est concentré à sec sous pression réduite à 30°C. On obtient ainsi après battage dans 40 ml d'acétate d'éthyle 9 g de chlorhydrate d'amino-3 mercapto-2 phényl-3 propionate de méthyle-(2RS,3S) sous forme d'un solide ocre, utilisé tel quel dans les synthèses ultérieures.
F. Le tert-butoxycarbonylamino-3 mercapto-2 phényl-3 propionate de méthyle-(2RS,3S) peut être préparé de la manière suivante : à une solution de 39 g de tert-butoxycarbonylamino-3 acétylthio-2 phényl-3 propionate de méthyle-(2RS,3S) dans 400 ml de méthanol on ajoute 8,2 g de méthylate de sodium. Le mélange réactionnel est agité pendant 3 heures à une température voisine de 25°C puis concentré à sec sous pression réduite à 30°C. Le résidu obtenu est dilué par 200 ml d'acétate d'éthyle et 200 ml d'une solution aqueuse 1N d'acide chlorhydrique. La phase organique est séparée par décantation et la phase aqueuse est extraite par 2 fois 50 ml d'acétate d'éthyle. Les phases organiques réunies sont lavées par 50 ml d'une solution aqueuse saturée de chlorure de sodium, séchées sur sulfate de magnésium et concentrées à sec sous pression réduite à 40°C. On obtient ainsi 38,0 g de tert-butoxycarbonylamino-3 mercapto-2 phényl-3 propionate de méthyle-(2RS,3S) sous forme d'une huile jaune utilisée telle quelle dans les synthèses ultérieures.
G. Le tert-butoxycarbonylamino-3 acétylthio-2 phényl-3 propionate de méthyle-(2RS,3S) peut être préparé de la manière suivante : à une solution de 50 g de tert-butoxycarbonylamino-3 tosyloxy-2 phényl-3 propionate de méthyle (2R, 3S) dans 400 ml d'acétone on ajoute 28,2 g de thioacétate de potassium. Le milieu réactionnel est chauffé au reflux pendant 4 heures puis évaporé à sec sous pression réduite à 30°C. Le résidu obtenu est dilué par 200 ml d'acétate d'éthyle et 200 ml d'eau. La phase organique est séparée par décantation, lavée par 2 fois 100 ml d'eau et par 100 ml d'une solution aqueuse saturée de chlorure de sodium, séchée sur sulfate de magnésium et concentrée à sec sous pression réduite à 40°C. On obtient ainsi 41,0 g de tert-butoxycarbonylamino-3 acétylthio-2 phényl-3 propionate de méthyle-(2RS,3S) sous forme d'une huile brune utilisée telle quelle dans les synthèses ultérieures.
H. Le tert-butoxycarbonylamino-3 tosyloxy-2 phényl-3 propionate de méthyle-(2R,3S) peut être préparé de la manière suivante : à un mélange de 100 g de tert-butoxycarbonylamino-3 hydroxy-2 phényl-3 propionate de méthyle-(2R,3S) et 72 ml de triéthylamine dans 500 ml de dichlorométhane on ajoute à une température voisine de 5°C une solution de 71 g de chlorure de tosyle dans 500 ml de dichlorométhane. Après retour à une température voisine de 20°C le milieu réactionnel est agité 12 heures puis concentré à sec sous pression réduite à 30°C. Le résidu obtenu est dilué par 500 ml d'acétate d'éthyle et 500 ml d'eau. La phase organique est séparée par décantation, lavée par 2 fois 200 ml d'eau et par 200 ml d'une solution aqueuse saturée de chlorure de sodium, séchée sur sulfate de magnésium et concentrée à sec sous pression réduite à 40°C. On obtient ainsi 148,0 g de tert-butoxycarbonylamino-3 tosyloxy-2 phényl-3 propionate de méthyle-(2R,3S) sous forme d'un solide blanc fondant à 120°C.

Le tert-butoxycarbonylamino-3 hydroxy-2 phényl-3 propionate de méthyle-(2R,3S) peut être préparé selon le mode opératoire décrit dans le brevet WO 93/17997.

Le dimorpholino-2,2 acétate de tert-butyle peut être préparé selon le mode opératoire décrit par R. HEYMES et coll., Bull. Soc. Chim. Fr, 2343-9 (1973).

### EXEMPLE 2

On opère d'une manière analogue à celle décrite à l'exemple 1 mais à partir d'une solution contenant 0,7 g d'(amino-2 acétyl)-3 méthoxycarbonyl-5 phényl-4 thiazolidinecarboxylate-2 de tert-butyle-(2S,4S,5S) dans 20 ml de tétrahydrofuranne et de 250 µl d'isocyanate de méthyl-3 phényle. Le produit brut est purifié par chromatographie sur silice [éluant : acétate d'éthyle-cyclohexane (20/80 en volumes)]. Les fractions contenant le produit attendu sont réunies et concentrées à sec sous pression réduite à 40°C. On obtient ainsi, 0,28 g de méthoxycarbonyl-5 {[(méthyl-3 phényl)-3 uréido]-2 acétyl}-3 phényl-4 thiazolidinecarboxylate-2 de tert-butyle-(2S,4S,5S) sous forme d'une meringue blanche, Rf = 0,39 [acétate d'éthyle-cyclohexane (50/50 en volumes)]; R.M.N. ¹H (200 MHz, (CD₃)₂SO avec ajout de quelques gouttes de CD₃COOD, à une température de 297 K, δ en ppm): 1,48 (s, 9H : CH₃ du tert-butyle); 2,21 (s, 3H : ArCH₃); 3,28 et 4,15 (respectivement d large et d, J = 17,5 Hz, 1H chacun : COCH₂N); 3,37 (s, 3H : COOCH₃); 5,10 (d, J = 7 Hz, 1H : H en C5 du cycle); 5,43 (s, 1H : H en C2 du cycle); 5,68 (d, J = 7 Hz, 1H : H en C4 du cycle); 6,70 (d large, J = 7,5 Hz, 1H : H aromatique (H en ortho du CH₃)); 7,06 (t, J = 7,5 Hz, 1H : H aromatique (H en méta du CH₃)); de 7,00 à 7,50 (mt, 5H : H aromatiques); 7,72 (mt, 2H : H ortho du phényle).
A. L'(amino-2 acétyl)-3 méthoxycarbonyl-5 phényl-4 thiazolidinecarboxylate-2 de tert-butyle-(2S,4S,5S) peut être préparé d'une manière analogue à celle décrite à l'exemple 1A mais à partir de 0,77 g d'(azido-2 acétyl)-3 méthoxycarbonyl-5 phényl-4 thiazolidinecarboxylate-2 de tert-butyle(25,45,55, de 0,50 g de triphénylphosphine et de 34 µl d'eau. Après agitation pendant 2 heures à une température voisine de 25°C la solution brute obtenue contenant environ 0,7 g d'(amino-2 acétyl)-3 méthoxycarbonyl-5 phényl-4 thiazolidinecarboxylate-2 de tert-butyle-(2S,4S,5S) est utilisée telle quelle dans les synthèses ultérieures.
B. L'(azido-2 acétyl)-3 méthoxycarbonyl-5 phényl-4 thiazolidinecarboxylate-2 de tert-butyle-(2S,4S,5S) peut être préparé d'une manière analogue à celle décrite à l'exemple 1B mais à partir de 0,58 g de (bromo-2 acétyl)-3 méthoxycarbonyl-5 phényl-4 thiazolidinecarboxylate-2 de tert-butyle-(2S,4S,5S) et de 0,09 g d'azoture de sodium. On obtient ainsi 0,5 g d'(azido-2 acétyl)-3 méthoxycarbonyl-5 phényl-4 thiazolidinecarboxylate-2 de tert-butyle-(2S,4S,5S) sous forme d'une huile jaune utilisée telle quelle dans les synthèses ultérieures.

### EXEMPLE 3

A une solution de 0,13 g de méthoxycarbonyl-5 {[(méthyl-3 phényl)-3 uréido]-2 acétyl}-3 phényl-4 thiazolidinecarboxylate-2 de tert-butyle-(2S,4S,5S) dans 10 ml de tétrahydrofuranne on ajoute à une température voisine de 20°C, 2,5 ml d'une solution aqueuse 0,1N d'hydroxyde de sodium. Le milieu réactionnel est agité pendant 12 heures à cette température, puis est dilué par 20 ml d'eau et lavé par 2 fois 20 ml d'oxyde de diéthyle. La phase aqueuse décantée est amenée à pH 2 par addition d'une solution aqueuse 1N d'acide sulfurique et extraite par 3 fois 20 ml d'oxyde de diéthyle. Les phases organiques réunies sont séchées sur sulfate de magnésium et concentrées à sec sous pression réduite à 30°C. On obtient 0,1 g de meringue que l'on dissout dans 2,0 ml d'une solution aqueuse 0,1N d'hydroxyde de sodium. L'insoluble est séparé par filtration et la phase aqueuse est amenée à pH 2 par addition d'une solution aqueuse 1N d'acide sulfurique. Le produit précipité est séparé par filtration, lavé par 2 fois 2 ml d'eau et séché à l'air. On obtient ainsi 0,07 g d'acide tert-butoxycarbonyl-2 {[(méthyl-3 phényl)-3 uréido]-2 acétyl}-3 phényl-4 thiazolidinecarboxylique-5-(2S,4S,5R) sous forme d'un solide blanc fondant à 130°C, [α]_{D}²⁵ = -5,1° ± 1,0° (c = 0,49; méthanol); R.M.N. ¹H (200 MHz, (CD₃)₂SO avec ajout de quelques gouttes de CD₃COOD, à une température de 393 K, δ en ppm): 1,51 (s, 9H : CH₃ du tert-butyle); 2,26 (s, 3H : ArCH₃); 3,70 et 4,05 (respectivement d large et d, J = 17 Hz, 1H chacun : COCH₂N); 4,21 (d, J = 4 Hz, 1H:H en C5 du cycle); 5,73 (s, 1H : H en C2 du cycle); 5,79 (d, J = 4 Hz, 1H:H en C4 du cycle); 6,76 (d large, J = 7,5 Hz, 1H : H aromatique (H en ortho du CH₃)); 7,10 (t, J = 7,5 Hz, 1H : H aromatique (H en méta du CH₃)); de 7,10 à 7,50 (mt, 5H : H aromatiques); 7,72 (dd, J = 7,5 et 2 Hz, 2H : H ortho du phényle).

### EXEMPLE 4

Dans un ballon contenant 0,3 g d'acide tert-butoxycarbonyl-2 {[(benzyloxycarbonylméthyl-3 phényl)-3 uréido]-2 acétyl}-3 phényl-4 thiazolidinecarboxylique-5-(2S,4S,5R), 0,18 g de formiate d'ammonium et 0,3 g de palladium sur charbon à 10 %, on ajoute lentement sous atmosphère inerte 10 ml de méthanol. Le milieu réactionnel est chauffé à reflux pendant 1 heure, puis refroidi à une température voisine de 25°C. Le catalyseur est séparé par filtration et le filtrat est concentré à sec sous pression réduite à 40°C. Le résidu obtenu est dissous dans 9,4 ml d'une solution aqueuse 0,1N d'hydroxyde de sodium. La solution aqueuse obtenue est filtrée, amenée à pH 2 par addition d'une solution aqueuse d'acide chlorhydrique 0,1 N. Le produit précipité est séparé par filtration, lavé par 2 fois 5 ml d'eau et séché à l'air. On obtient ainsi, 0,19 g d'acide {[(tert-butoxycarbonyl-2 carboxy-5 phényl-4 thiazolidinyl-3-(2S,4S,5R))-2 oxo-2 éthyl]-3 uréido}-3 phénylacétique sous forme d'un solide blanc fondant à 130°C, [α]_{D}²⁵ = -3,9° ± 1,0 (c = 0,407; MeOH); R.M.N. ¹H (200 MHz, (CD₃)₂SO avec ajout de quelques gouttes de CD₃COOD, à une température de 373 K, δ en ppm): 1,49 (s, 9H : CH₃ du tert-butyle); 3,49 (s, 2H : ArCH₂COO); 3,65 et 4,03 (respectivement d large et d, J = 17 Hz, 1H chacun : COCH₂N); 4,19 (d, J = 3,5 Hz, 1H : H en C5 du cycle); 5,70 (s, 1H : H en C2 du cycle); 5,76 (d, J = 3,5 Hz, 1H : H en C4 du cycle); 6,83 (d large, J = 7,5 Hz, 1H : H aromatique (H en ortho du CH₂)); 7,14 (t, J = 7,5 Hz, 1H : H aromatique (H en méta du CH₂); de 7,20 à 7,50 (mt, 5H : H aromatiques); 7,72 (d large, J = 7,5 Hz, 2H : H ortho du phényle).
A. L'acide tert-butoxycarbonyl-2 {[(benzyloxycarbonylméthyl-3 phényl)-3 uréido]-2 acétyl}-3 phényl-4 thiazolidinecarboxylique-5-(2S,4S,5R) peut être préparé d'une manière analogue à celle décrite à l'exemple 3 mais à partir de 0,65 g de tert-butoxycarbonyl-2 {[(benzyloxycarbonylméthyl-3 phényl)-3 uréido]-2 acétyl}-3 phényl-4 thiazolidinecarboxylate-5 de méthyle-(2S,4S,5R) et de 7 ml d'une solution aqueuse 0,1N d'hydroxyde de sodium dans 10 ml de tétrahydrofuranne. On obtient ainsi 0,37 g d'acide tert-butoxycarbonyl-2 {[(benzyloxycarbonylméthyl-3 phényl)-3 uréido]-2 acétyl}-3 phényl-4 thiazolidinecarboxylique-5-(2S,4S,5R) sous forme d'un solide blanc fondant à 114°C.
B. Le tert-butoxycarbonyl-2 {[(benzyloxycarbonylméthyl-3 phényl)-3 uréido]-2 acétyl}-3 phényl-4 thiazolidinecarboxylate-5 de méthyle-(2S,4S,5R) peut être préparé d'une manière analogue à celle décrite à l'exemple 1 mais à partir de 0,57 g d'(amino-2 acétyl)-3 méthoxycarbonyl-5 phényl-4 thiazolidinecarboxylate-2 de tert-butyle-(2S,4S,5R) et de 0,5 g d'isocyanato-3 phénylacétate de benzyle. Le produit brut est purifié par chromatographie sur silice. [éluant : acétate d'éthyle-cyclohexane (40/60 en volumes)]. Les fractions contenant le produit attendu sont réunies et concentrées à sec sous pression réduite à 30°C. On obtient ainsi 0,3 g de tert-butoxycarbonyl-2 {[(benzyloxycarbonylméthyl-3 phényl)-3 uréido]-2 acétyl}-3 phényl-4 thiazolidinecarboxylate-5 de méthyle-(2S,4S,5R) sous forme d'un produit amorphe utilisé tel quel dans les synthèses ultérieures.
C. L'isocyanato-3 phénylacétate de benzyle peut être préparé de la manière suivante : à une suspension de 0,5 g de charbon dans un mélange de 2,6 ml de chloroformiate de trichlorométhyle et de 75 ml de toluène, on ajoute en 15 minutes à une température voisine de -30°C, une solution de 5,0 g d'amino-3 phénylacétate de benzyle dans 40 ml de toluène. Le mélange réactionnel est agité pendant 2 heures, puis chauffé au reflux pendant 3 heures. Après refroidissement à une température voisine de 25°C, le milieu réactionnel est dégazé par barbottage d'azote; le catalyseur est séparé par filtration et le filtrat est concentré à sec sous pression réduite à 40°C. On obtient ainsi 6,4 g d'isocyanato-3 phénylacétate de benzyle sous forme d'une huile jaune utilisée telle quelle dans les synthèses ultérieures.
D. L'amino-3 phénylacétate de benzyle peut être préparé de la manière suivante : à un mélange de 28 g de nitro-3 phénylacétate de benzyle dans 125 ml de méthanol et 1300 ml d'eau, on ajoute 265 g de chlorure d'ammonium et 130 g de zinc en poudre. Le milieu réactionnel est chauffé au reflux pendant 1 heure puis refroidi à une température voisine de 0°C. Les sels insolubles sont séparés par filtration et le filtrat est extrait par 3 fois 500 ml d'oxyde de diéthyle. Les phases organiques collectées sont lavées successivement par 100 ml d'eau et 200 ml d'une solution aqueuse saturée de chlorure de sodium. On obtient ainsi, après séchage sur sulfate de magnésium et concentration à sec sous pression réduite, 20,5 g d'amino-3 phénylacétate de benzyle sous forme d'une huile jaune utilisée telle quelle dans les synthèses ultérieures.
E. Le nitro-3 phénylacétate de benzyle peut être préparé de la manière suivante : à un mélange contenant 21,0 g d'acide nitro-3 phénylacétique et 0,5 ml de diméthylformamide dans 200 ml de dichloro-1,2 éthane, on ajoute lentement 10,3 ml de dichlorure d'oxalyle. Le milieu réactionnel est agité pendant 3 heures à une température voisine de 25°C, puis on ajoute 12,5 g d'alcool benzylique. L'agitation est poursuivie pendant 12 heures à cette même température, puis le milieu réactionnel est lavé par 2 fois 100 ml d'une solution aqueuse saturée d'hydrogénocarbonate de sodium, 100 ml d'eau et 100 ml d'une solution aqueuse saturée de chlorure de sodium. La phase organique collectée est séchée sur sulfate de magnésium et concentrée à sec sous pression réduite à 40°C. Le résidu obtenu est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi, 28,0 g de nitro-3 phénylacétate de benzyle sous forme d'une huile jaune utilisée telle quelle dans les synthèses ultérieures.

### EXEMPLE 5

On opère comme à l'exemple 3 mais à partir de 0,3 g de {{[(benzyloxycarbonyléthyl-1)-3 phényle(S)]-3 uréido}-2 acétyl}-3 tert-butoxycarbonyl-2 phényl-4 thiazolidinecarboxylate-5 de méthyle-(2S,4S,5S), et de 4,5 ml d'une solution aqueuse 0,1N d'hydroxyde de sodium. On obtient ainsi 0,15 g d'acide {{[(benzyloxycarbonyléthyl-1)-3 phényl-(S)]-3 uréido}-2 acétyl}-3 tert-butoxycarbonyl-2 phényl-4 thiazolidinecarboxylique-5-(2S,4S,5R) sous forme d'un solide blanc fondant à 96°C, [α]_{D}²⁵ = + 6° ± 1° (c = 0,46; MeOH); R.M.N. ¹H (250 MHz, (CD₃)₂SO avec ajout de quelques gouttes de CD₃COOD, à une température de 393 K, δ en ppm): 1,45 (d, J=7,5 Hz, 3H : CH₃); 1,51 (s, 9H : CH₃ du tert-butyle); 3,66 et 4,05 (respectivement d large et d, J =17,5 Hz, 1H chacun : COCH₂N); 3,77 (q, J = 7,5 Hz, 1H : CHCOO); 4,19 (d, J = 4 Hz, 1H : H en C5 du cycle); 5,12 (s, 2H : OCH₂); 5,71 (s, 1H : H en C2 du cycle); 5,77 (d, J = 4 Hz, 1H : H en C4 cycle); 6,85 (d large, J =7,5 Hz, 1H : H aromatique (H en ortho du CH)); 7,15 (t, J = 7,5 Hz, 1H : H aromatique (H en méta du CH); de 7,20 à 7,50 (mt, 10H: H aromatiques); 7,72 (d large, J = 7,5 Hz, 2H : H ortho du phényle en 4).
A. Le {{[(benzyloxycarbonyléthyl-1)-3 phényl-(S)]-3 uréido}-2 acétyl}-3 tert-butoxycarbonyl-2 phényl-4 thiazolidinecarboxylate-5 de méthyle(25,45,55) peut être préparé d'une manière analogue à celle décrite à l'exemple 1 mais à partir de 0,50 g d'(amino-2 acétyl)-3 méthoxycarbonyl-5 phényl-4 thiazolidinecarboxylate-2 de tert-butyle-(2S,4S,5S) et de 0,5 g d'(isocyanato-3 phényl)-2 propionate de benzyle-(S). Le produit brut est purifié par chromatographie sur silice [éluant : acétate d'éthyle-cyclohexane (40/60 en volumes)]. Les fractions contenant le produit attendu sont réunies et concentrées à sec sous pression réduite à 30°C. On obtient ainsi 0,31 g de {{[(benzyloxycarbonyléthyl-1)-3 phényl-(S)]-3 uréido}-2 acétyl}-3 tert-butoxycarbonyl-2 phényl-4 thiazolidinecarboxylate-5 de méthyle-(2S,4S,5S) sous forme d'un produit amorphe utilisé tel quel dans les synthèses ultérieures.
B. L'(isocyanato-3 phényl)-2 propionate de benzyle-(S) peut être préparé d'une manière analogue à celle décrite à l'exemple 4C mais à partir de 2,85 g d'(amino-3 phényl)-2 propionate de benzyle-(S), de 0,24 g de charbon et de 1,5 ml de chloroformiate de trichlorométhyle. On obtient ainsi 3,1 g d'(isocyanato-3 phényl)-2 propionate de benzyle-(S) sous forme d'une huile jaune utilisée telle quelle dans les synthèses ultérieures.
C. L'(amino-3 phényl)-2 propionate de benzyle-(S) peut être préparé d'une manière analogue à celle décrite à l'exemple 4D mais à partir de 8 g de (nitro-3 phényl)-2 propionate de benzyle-(S), de 75 g de chlorure d'ammonium et de 37 g de zinc en poudre. On obtient ainsi 6,7 g d'(amino-3 phényl)-2 propionate de benzyle-(S) sous forme d'une huile jaune utilisée telle quelle dans les synthèses ultérieures.
D. Le (nitro-3 phényl)-2 propionate de benzyle-(S) peut être préparé d'une manière analogue à celle décrite à l'exemple 4E mais à partir de 9,75 g d'acide (nitro-3 phényl)-2 propionique-(S), de 4,7 ml de dichlorure d'oxalyle, de 0,5 ml de diméthylformamide et de 5,4 g d'alcool benzylique. Le produit brut est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi, 11,5 g de (nitro-3 phényl)-2 propionate de benzyle-(S) sous forme d'une huile jaune utilisée telle quelle dans les synthèses ultérieures.

L'acide (nitro-3 phényl)-2 propionique-(S) peut être préparé selon le mode opératoire décrit par F. Nerdel et H. Härter, Liebigs Ann. Chem., 621, 22-33 (1959).

### EXEMPLE 6

On opère comme à l'exemple 4 mais à partir de 0,58 g d'acide {{[(benzyloxycarbonyléthyl-1)-3 phényl-(S)]-3 uréido}-2 acétyl}-3 tert-butoxycarbonyl-2 phényl-4 thiazolidinecarboxylique-5-(2S,4S,5R), de 0,34 g de formiate d'ammonium et 0,6 g de palladium sur charbon à 10%. On obtient ainsi, 0,21 g d'acide {{[(tert-butoxycarbonyl-2 carboxy-5 phényl-4 thiazolidinyl-3-(2S, 4S, 5R))-2 oxo-2 éthyl]-3 uréido}-3 phényl}-2 propionique-(S) sous forme d'un solide blanc fondant à 142°C, [α]_{D}²⁵ = + 24,5° ± 0,6° (c = 1,01; MeOH); R.M.N. ¹H (200 MHz, (CD₃)₂SO avec ajout de quelques gouttes de CD₃COOD, à une température de 373 K, δ en ppm): 1,41 (d, J = 7 Hz, 3H : CH₃); 1,51 (s, 9H : CH₃ du tert-butyle); 3,62 (q, J = 7 Hz, 1H : CHCOO); 3,66 et 4,05 (respectivement d large et d, J = 17,5 Hz, 1H chacun : COCH₂N); 4,18 (d, J = 4 Hz, 1H : H en C5 du cycle); 5,72 (s, 1H : H en C2 du cycle); 5,77 (d, J = 4 Hz, 1H : H en C4 du cycle); 6,86 (d large, J = 7,5 Hz, 1H : H aromatique (H en ortho du CH)); 7,17 (t, J = 7,5 Hz, 1H : H aromatique (H en méta du CH); de 7,20 à 7,50 (mt, 5H : H aromatiques); 7,73 (dd, J = 7,5 et 1,5 Hz, 2H : H ortho du phényle).

### EXEMPLE 7

On opère d'une manière analogue à celle décrite dans l'exemple 1 mais à partir de 0,30 g d'(amino-2 acétyl)-3 méthoxycarbonyl-5 (fluoro-2 phényl)-4 thiazolidinecarboxylate-2 de tert-butyle-(2S,4S,5S) en solution dans 10 ml de tétrahydrofuranne et de 100 µl d'isocyanate de méthyl-3 phényle. Le produit brut est purifié par chromatographie sur silice [éluant: acétate d'éthyle-cyclohexane (30/70 en volumes)]. Les fractions contenant le produit attendu sont réunies et concentrées à sec sous pression réduite à 40°C. On obtient ainsi, 0,08 g de méthoxycarbonyl-5 {[(méthyl-3 phényl)-3 uréido]-2 acétyl}-3 (fluoro-2 phényl)-4 thiazolidinecarboxylate-2 de tert-butyle-(2S,4S,5S) sous forme de meringue jaune, Rf = 0,43, [acétate d'éthyle-cyclohexane (40/60 en volumes); R.M.N. ¹H (200 MHz, (CD₃)₂SO avec ajout de quelques gouttes de CD₃COOD, à une température de 393 K, δ en ppm): 1,52 (s, 9H : CH₃ du tert-butyle); 2,25 (s, 3H : ArCH₃); 3,34 (s, 3H : COOCH₃); 3,70 et 4,05 (respectivement d large et d, J = 17 Hz, 1H chacun : COCH₂N); 4,95 (d, J = 7,5 Hz, 1H : H en C5 du cycle); 5,75 (s, 1H : H en C2 du cycle); 6,05 (d, J= 7,5 Hz, 1H : H en C4 du cycle); 6,76 (d large, J = 7,5 Hz, 1H : H aromatique (H en ortho du CH₃)); 7,09 (t, J = 7,5 Hz, 1H : H aromatique (H en méta du CH₃)); de 7,10 à 7,50 (mt, 5H : H aromatiques); 8,00 (mt, 1H : H aromatique (H en méta du F)).
A. L'(amino-2 acétyl)-3 méthoxycarbonyl-5 (fluoro-2 phényl)-4 thiazolidinecarboxylate-2 de tert-butyle-(2S,4S,5S) peut être préparé d'une manière analogue à celle décrite à l'exemple 1A mais à partir de 0,5 g d'(azido-2 acétyl)-3 méthoxycarbonyl-5 (fluoro-2 phényl)-4 thiazolidinecarboxylate-2 de tert-butyle-(2S,4S,5S), de 0,20 g de triphénylphosphine et de 14 µl d'eau. La solution brute obtenue contenant environ 0,30 g d'(amino-2 acétyl)-3 méthoxycarbonyl-5 (fluoro-2 phényl)-4 thiazolidinecarboxylate-2 de tert-butyle-(2S,4S,5S) est utilisée telle quelle dans les synthèses ultérieures.
B. L'(azido-2 acétyl)-3 méthoxycarbonyl-5 (fluoro-2 phényl)-4 thiazolidinecarboxylate-2 de tert-butyle-(2S,4S,5S) peut être préparé d'une manière analogue à celle décrite à l'exemple 1B mais à partir de 0,35 g de (bromo-2 acétyl)-3 méthoxycarbonyl-5 (fluoro-2 phényl)-4 thiazolidinecarboxylate-2 de tert-butyle-(2S,4S,5S) et de 0,05 g d'azoture de sodium. On obtient ainsi 0,5 g d'(azido-2 acétyl)-3 méthoxycarbonyl-5 (fluoro-2 phényl)-4 thiazolidinecarboxylate-2 de tert-butyle-(2S,4S,5S) sous forme d'une huile jaune utilisée telle quelle dans les synthèses ultérieures.
C. Le (bromo-2 acétyl)-3 méthoxycarbonyl-5 (fluoro-2 phényl)-4 thiazolidinecarboxylate-2 de tert-butyle-(2S,4S,5S) peut être préparé d'une manière analogue à celle décrite à l'exemple 1C mais à partir de 0,8 g de méthoxycarbonyl-5 (fluoro-2 phényl)-4 thiazolidinecarboxylate-2 de tert-butyle-(2RS,4S,5S), de 0,3 ml de triéthylamine et de 0,47 g de bromure de bromoacétyle. Le produit brut obtenu est purifié par chromatographie sur silice [éluant : acétate d'éthyle-cyclohexane (30/70 en volumes)]. Les fractions contenant le produit sont réunies et concentrées à sec sous pression réduite à 30°C. On obtient ainsi 0,35 g de (bromo-2 acétyl)-3 méthoxycarbonyl-5 (fluoro-2 phényl)-4 thiazolidinecarboxylate-2 de tert-butyle-(2S,4S,5S) sous forme d'une huile jaune utilisée telle quelle dans les synthèses ultérieures.
D. Le méthoxycarbonyl-5 (fluoro-2 phényl)-4 thiazolidinecarboxylate-2 de tert-butyle-(2RS,4S,5S) peut être préparé d'une manière analogue à celle décrite à l'exemple 1D mais à partir de 1,0 g de chlorhydrate d'amino-3 mercapto-2 (fluoro-2 phényl)-3 propionate de méthyle-(2S, 3S), de 1,0 ml de triéthylamine et de 2,1 g de dimorpholino-2,2 acétate de tert-butyle. Le produit brut obtenu est purifié par chromatographie sur silice [éluant : acétate d'éthyle-cyclohexane (20/80 en volumes)]. On obtient ainsi 0,8 g de méthoxycarbonyl-5 (fluoro-2 phényl)-4 thiazolidinecarboxylate-2 de tert-butyle-(2RS,4S,5S) sous forme d'une huile jaune utilisée telle quelle dans les synthèses ultérieures.
E. Le chlorhydrate d'amino-3 mercapto-2 (fluoro-2 phényl)-3 propionate de méthyle-(2S, 3S) peut être préparé d'une manière analogue à celle décrite à l'exemple 1E mais à partir de 8,2 g de tert-butoxycarbonylamino-3 mercapto-2 (fluoro-2 phényl)-3 propionate de méthyle-(2RS, 3S) et de 20 ml d'une solution méthanolique d'acide chlorhydrique 5N. L'agitation est poursuivie pendant 3 h à une température voisine de 20°C, puis le milieu réactionnel est concentré à sec sous pression réduite à 30°C. Le résidu obtenu est battu dans 20 ml d'acétate d'éthyle. Le solide obtenu est séparé par filtration et séché à l'air. On obtient ainsi 2,3 g de chlorhydrate d'amino-3 mercapto-2 phényl-3 propionate de méthyle-(2S, 3S) sous forme d'un solide blanc fondant à 172°C. On isole également après évaporation du filtrat 1,8 g de chlorhydrate d'amino-3 mercapto-2 phényl-3 propionate de méthyle-(2RS, 3S) sous forme d'un solide blanc utilisé tel quel dans les synthèses ultérieures.
F. Le tert-butoxycarbonylamino-3 mercapto-2 (fluoro-2 phényl)-3 propionate de méthyle-(2RS, 3S) peut être préparé d'une manière analogue à celle décrite à l'exemple 1F mais à partir de 9,8 g de tert-butoxycarbonylamino-3 acétylthio-2 phényl-3 propionate de méthyle (2RS, 3S) et de 1,35 g de méthylate de sodium. On obtient ainsi 8,2 g de tert-butoxycarbonylamino-3 mercapto-2 (fluoro-2 phényl)-3 propionate de méthyle-(2RS, 3S) sous forme d'une huile rouge utilisée telle quelle dans les synthèses ultérieures.
G. Le tert-butoxycarbonylamino-3 acétylthio-2 (fluoro-2 phényl)-3 propionate de méthyle-(2RS, 3S) peut être préparé d'une manière analogue à celle décrite à l'exemple 1G mais à partir de 30 g de tert-butoxycarbonylamino-3 tosyloxy-2 (fluoro-2 phényl)-3 propionate de méthyle (2R, 3S) et de 17,2 g de thioacétate de potassium. On obtient ainsi 9,8 g de tert-butoxycarbonylamino-3 acétylthio-2 phényl-3 propionate de méthyle (2RS, 3S) sous forme d'une huile rouge utilisée telle quelle dans les synthèses ultérieures.
H. Le tert-butoxycarbonylamino-3 tosyloxy-2 (fluoro-2 phényl)-3 propionate de méthyle-(2R, 3S) peut être préparé d'une manière analogue à celle décrite à l'exemple 1H mais à partir de 20 g de tert-butoxycarbonylamino-3 hydroxy-2 (fluoro-2 phényl)-3 propionate de méthyle-(2R, 3S), de 13,4 ml de triéthylamine et de 13,3 g de chlorure de tosyle. On obtient ainsi 30,8 g de tert-butoxycarbonylamino-3 tosyloxy-2 (fluoro-2 phényl)-3 propionate de méthyle-(2R, 3S) sous forme d'un solide blanc fondant à 110°C et utilisé tel quel dans les synthèses ultérieures.
I. Le tert-butoxycarbonylamino-3 hydroxy-2 (fluoro-2 phényl)-3 propionate de méthyle-(2R,3S) peut être préparé de la manière suivantes : à 2 g d'une suspension de palladium à 5% sur du charbon activé en poudre dans 10 ml d'éthanol, on ajoute une solution de 50 g d'hydroxy-2 ((phényl-1 éthylamino-(S))-3 (fluoro-2 phényl)-3 propionate de méthyle-(2R,3S) dans un mélange de 200 ml de méthanol et 20 ml d'acide acétique. Le mélange réactionnel est agité pendant 8 heures sous atmosphère d'hydrogène (150 kPa) à une température voisine de 25°C. Le catalyseur est séparé par filtration et lavé par 2 fois 50 ml de méthanol. A la solution méthanolique ainsi obtenue d'amino-3 hydroxy-2 (fluoro-2 phényl)-3 propionate de méthyle-(2R,3S) on ajoute, à une température voisine de 5°C, 27 ml d'une solution aqueuse d'hydroxyde de sodium à 30% puis, après retour à une température voisine de 25°C, une solution de 34,2 g de dicarbonate de di-tert-butyle dans 70 ml de méthanol et 100 ml d'eau. Le milieu réactionnel est agité pendant 12 heures puis refroidi à une température voisine de 5°C et dilué par 200 ml d'eau. Le milieu hétérogène obtenu est battu pendant 1 heure à cette température et le solide est séparé par filtration. On obtient ainsi après séchage 39,6 g de tert-butoxycarbonylamino-3 hydroxy-2 (fluoro-2 phényl)-3 propionate de méthyle-(2R,3S) sous forme d'un solide blanc fondant à 95°C, [α]_{D}²⁵ = - 6,3° ± 0,9° (C = 0,627, MeOH).
J. L'hydroxy-2 ((phényl-1 éthylamino-(S))-3 (fluoro-2 phényl)-3 propionate de méthyle-(2R,3S) peut être préparé de la manière suivante : à une suspension de 50 g d'hydroxy-3 (fluoro-2 phényl)-4 (phényl-1 éthyl-(S))-1 azétidinone-2-(3R,4S) dans 120 ml de méthanol refroidie à une température voisine de 5°C on ajoute lentement 47,5 ml d'une solution méthanolique 8N d'acide chlorhydrique en maintenant la température en dessous de 20°C. Le mélange réactionnel est agité pendant 12 heures à cette température, refroidi à une température voisine de 5°C et alcalinisé jusqu'à pH 7 par addition lente de 35 ml d'une solution aqueuse à 30 % d'hydroxyde de sodium. On ajoute 700 ml d'eau à une température voisine de 0°C. L'agitation est poursuivie pendant 1 heure puis le solide obtenu est séparé par filtration, lavé par 2 fois 100 ml d'eau refroidi à une température voisine de 5°C et séché à l'air. On obtient ainsi 53,6 g d'hydroxy-2 ((phényl-1 éthylamino-(S))-3 (fluoro-2 phényl)-3 propionate de méthyle-(2R,3S) sous forme d'un solide blanc, fondant à une température inférieure à 50°C et utilisé tel quel dans les synthèses ultérieures.
K. L'hydroxy-3 (fluoro-2 phényl)-4 (phényl-1 éthyl-(S))-1 azétidinone-2-(3R,4S) peut être préparée de la manière suivante : à une solution, refroidie à une température voisine de 0°C, de 110 g d'un mélange en proportion molaire 65/35 des deux diastéréoisomères d'acétoxy-3 (fluoro-2 phényl)-4 (phényl-1 éthyl-(S))-1 azétidinone-2 formes A et B dans 600 ml de méthanol on ajoute 11 ml d'une solution aqueuse à 30 % d'hydroxyde de sodium. Le milieu réactionnel est agité pendant 1 heure à une température voisine de 0°C, dilué par addition de 250 ml d'eau et 60 ml de méthanol. L'agitation est poursuivie pendant 2 heures à cette température puis le solide est séparé par filtration, lavé par 50 ml d'eau refroidie à une température voisine de 5°C et séché à l'air. On obtient ainsi 50,5 g d'hydroxy-3 (fluoro-2 phényl)-4 (phényl-1 éthyl-(S))-1 azétidinone-2-(3R,4S) sous forme d'un solide blanc fondant à 138°C.
L. Le mélange des formes A et B des deux diastéréoisomères d'acétoxy-3 (fluoro-2 phényl)-4 (phényl-1 éthyl-(S))-1 azétidinone-2 peut être préparé de la manière suivante : à un mélange contenant une solution de 190 g de phényl-1 N-(fluoro-2 benzylidène)-éthylamine-(S) dans 200 ml de toluène et 148 ml de N-éthyl-morpholine on ajoute goutte à goutte, en 2 heures et à une température voisine de 20°C, 84 ml de chlorure d'acétoxy-2 acétyle. Le mélange réactionnel est agité pendant 12 heures à cette température puis additionné de 500 ml d'une solution aqueuse 2,5 N d'acide chlorhydrique. La phase organique est séparée par décantation, lavée par 100 ml d'eau et par 2 fois 200 ml d'une solution aqueuse saturée d'hydrogénocarbonate de sodium, séchée sur sulfate de magnésium et concentrée à sec sous pression réduite à 40°C. On obtient ainsi 220 g d'un mélange en proportion molaire 65/35 des formes A et B des deux diastéréoisomères d'acétoxy-3 (fluoro-2 phényl)-4 (phényl-1 éthyl-(S))-1 azétidinone-2 sous forme d'une huile brune utilisée telle quelle dans les synthèse ultérieures.
M. La phényl-1 N-(fluoro-2 benzylidène)-éthylamine-(S) peut être préparée de la manière suivante : à une solution de 100 g de fluoro-2 benzaldéhyde dans 500 ml de toluène on ajoute 102 ml de phényl-1 éthylamine-(S). Le milieu réactionnel est laissé pendant 12 heures à une température voisine de 20°C. L'eau formée est séparée par décantation et la phase organique est séchée par distillation azéotropique pendant 2 heures à l'aide d'un appareil de Dean-Stark. On obtient ainsi, après évaporation des solvants, 189 g de phényl-1 N-(fluoro-2 benzylidène)-éthylamine-(S) sous forme d'une huile jaune utilisée telle quelle dans les synthèses ultérieures.

### EXEMPLE 8

On opère d'une manière analogue à celle décrite à l'exemple 1 mais à partir de 0,34 g d'(amino-2 acétyl)-3 méthoxycarbonyl-5 (fluoro-2 phényl)-4 thiazolidinecarboxylate-2 de tert-butyle-(2S,4S,5R) en solution dans 10 ml de tétrahydrofuranne et de 106 µl d'isocyanate de méthyl-3 phényle. Le produit brut est purifié par chromatographie sur silice [éluant : acétate d'éthyle-cyclohexane (20/80 en volumes)]. Les fractions contenant le produit attendu sont réunies et concentrées à sec sous pression réduite à 40°C. On obtient ainsi après battage dans l'oxyde de diisopropyle, 0,17 g de méthoxycarbonyl-5 {[(méthyl-3 phényl)-3 uréido]-2 acétyl}-3 (fluoro-2 phényl)-4 thiazolidinecarboxylate-2 de tert-butyle-(2S,4S,5R) sous forme de meringue jaune pâle, Rf = 0,30, [acétate d'éthyle-cyclohexane (30/70 en volumes); R.M.N. ¹H (250 MHz, (CD₃)₂SO, à une température de 363 K, δ en ppm): 1,53 (s, 9H : CH₃ du tert-butyle); 2,27 (s, 3H : ArCH₃); 3,34 (s, 3H : COOCH₃); de 3,60 à 3,80 et 4,06 (respectivement mt et dd (J = 17 et 5 Hz), 1H chacun : COCH₂N); 4,33 (d, J = 3 Hz, 1H : H en C5 du cycle); 5,74 (mt, 1H : H en C2 du cycle); 6,01 (d, J = 3Hz, lH : H en C4 du cycle); 6,23 (t, J = 5 Hz, 1H : NHCO); 6,78 (d large, J = 7,5 Hz, 1H : H aromatique (H en ortho du CH₃)); 7,09 (t, J = 7,5 Hz, 1H : H aromatique (H en méta du CH₃)); de 7,10 à 7,50 (mt, 5H : H aromatiques); 8,03 (mt, 1H : H aromatique (H en méta du F)); 8,40 (s, 1H : ArNHCO).
A. L'(amino-2 acétyl)-3 méthoxycarbonyl-5 (fluoro-2 phényl)-4 thiazolidinecarboxylate-2 de tert-butyle-(2S,4S,5R) peut être préparé d'une manière analogue à celle décrite à l'exemple lA mais à partir de 0,56 g d'(azido-2 acétyl)-3 méthoxycarbonyl-5 (fluoro-2 phényl)-4 thiazolidinecarboxylate-2 de tert-butyle-(2S,4S,5R), de 0,21 g de triphénylphosphine et de 15 µl d'eau. La solution brute obtenue contenant environ 0,34 g d'(amino-2 acétyl)-3 méthoxycarbonyl-5 (fluoro-2 phényl)-4 thiazolidinecarboxylate-2 de tert-butyle-(2S,4S,5R) est utilisée telle quelle dans les synthèses ultérieures.
B. L'(azido-2 acétyl)-3 méthoxycarbonyl-5 (fluoro-2 phényl)-4 thiazolidinecarboxylate-2 de tert-butyle-(2S,4S,5R) peut être préparé d'une manière analogue à celle décrite à l'exemple 1B mais à partir de 0,39 g de (bromo-2 acétyl)-3 méthoxycarbonyl-5 (fluoro-2 phényl)-4 thiazolidinecarboxylate-2 de tert-butyle-(2S,4S,5R) et de 0,055 g d'azoture de sodium. On obtient ainsi 0,56 g d'(azido-2 acétyl)-3 méthoxycarbonyl-5 (fluoro-2 phényl)-4 thiazolidinecarboxylate-2 de tert-butyle-(2S,4S,5R) sous forme d'une huile jaune utilisée telle quelle dans les synthèses ultérieures.
C. Le (bromo-2 acétyl)-3 méthoxycarbonyl-5 (fluoro-2 phényl)-4 thiazolidinecarboxylate-2 de tert-butyle-(2S,4S,5R) peut être préparé d'une manière analogue à celle décrite à l'exemple 1C mais à partir de 1,7 g de méthoxycarbonyl-5 (fluoro-2 phényl)-4 thiazolidinecarboxylate-2 de tert-butyle-(2RS,4S,SRS), de 0,7 ml de triéthylamine et de 1,0 g de bromure de bromoacétyle. Le mélange brut de 2 g des diastéréoisomères obtenus ((2S,4S,5R) et (2S,4S,5S)) est séparé par chromatographie liquide haute performance en utilisant une colonne préparative du type 500-PREPAK (WATERS) et comme phase mobile, un mélange acétate d'éthyle-cyclohexane (10/90 en volumes). On obtient ainsi 0,4 g de (bromo-2 acétyl)-3 méthoxycarbonyl-5 (fluoro-2 phényl)-4 thiazolidinecarboxylate-2 de tert-butyle-(2S,4S,5R) (élué le premier) et 0,61 g de (bromo-2 acétyl)-3 méthoxycarbonyl-5 (fluoro-2 phényl)-4 thiazolidinecarboxylate-2 de tert-butyle-(2S,4S,5S) sous forme de deux huiles jaunes utilisées telles quelles dans les synthèses ultérieures.
D. Le méthoxycarbonyl-5 (fluoro-2 phényl)-4 thiazolidinecarboxylate-2 de tert-butyle-(2RS,4S,5RS) peut être préparé d'une manière analogue à celle décrite à l'exemple 1D mais à partir de 1,8 g de chlohydrate d'amino-3 mercapto-2 (fluoro-2 phényl)-3 propionate de méthyle-(2RS, 3S), de 1,9 ml de triéthylamine et de 3,9 g de dimorpholino-2,2 acétate de tert-butyle. Le produit brut obtenu est purifié par chromatographie sur silice [éluant : acétate d'éthyle-cyclohexane (20/80 en volumes)]. On obtient ainsi 1,0 g de méthoxycarbonyl-5 (fluoro-2 phényl)-4 thiazolidinecarboxylate-2 de tert-butyle-(2RS,4S,5RS) sous forme d'une huile jaune utilisée telle quelle dans les synthèses ultérieures.

### EXEMPLE 9

On opère d'une manière analogue à celle décrite à l'exemple 3 mais à partir de 0,35 g de méthoxycarbonyl-5 {[(méthyl-3 phényl)-3 uréido]-2 acétyl}-3 (fluoro-2 phényl)-4 thiazolidinecarboxylate-2 de tert-butyle-(2S,4S,5S) en solution dans 10 ml de tétrahydrofuranne et de 6,6 ml d'une solution aqueuse 0,1 N d'hydroxyde de sodium. On obtient ainsi 0,24 g d'acide tert-butoxycarbonyl-2 {[(méthyl-3 phényl)-3 uréido]-2 acétyl}-3 (fluoro-2 phényl)-4 thiazolidinecarboxylique-5-(2S,4S,5R) sous forme d'un solide blanc fondant à 148°C, [α]_{D}²⁵ = -17° ± 1° (c = 0,504; MeOH); R.M.N. ¹H (200 MHz, (CD₃)₂SO avec ajout de quelques gouttes de CD₃COOD, à une température de 373 K, δ en ppm): 1,51 (s, 9H : CH₃ du tert-butyle); 2,25 (s, 3H : ArCH₃); 3,76 et 4,04 (respectivement d large et d, J = 18 Hz, ¹H chacun : COCH₂N); 4,18 (d, J = 3,5 Hz, 1H : H en C5 du cycle); 5,70 (s, 1H : H en C2 du cycle); 5,97 (d, J = 3,5 Hz, 1H : H en C4 du cycle); 6,73 (d large, J = 7,5 Hz, 1H : H aromatique (H en ortho du CH₃)); 7,06 (t, J = 7,5 Hz, 1H : H aromatique(H en méta du CH₃)); de 7,10 à 7,50 (mt, 5H : H aromatiques); 8,00 (mt, 1H : H aromatique en 6 (H en méta du F)).

Les médicaments selon l'invention sont constitués par un composé de formule (I) sous forme libre ou sous forme d'un sel pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vemis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthyléneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles dans le traitement et la prévention des désordres liés à la CCK et à la gastrine au niveau du système nerveux et de l'appareil gastrointestinal. Ces composés peuvent donc être utilisés dans le traitement et la prévention des psychoses, des troubles anxieux, de la dépression, de la neurodégénération, des attaques de panique, de la maladie de Parkinson, de la diskynésie tardive, du syndrome du colon irritable, de la pancréatite aiguë, des ulcères, des désordres de la motilité intestinale, de certaines tumeurs sensibles à la CCK, des troubles de la mémoire, dans le sevrage aux traitements chroniques et abus d'alcool ou de médicaments, comme constricteurs de la pupille de l'oeil, comme analgésiques, comme potentialisateurs de l'activité analgésique des médicaments analgésiques narcotiques et non narcotiques et comme régulateurs de l'appétit.

Les doses dépendent de l'effet recherché, de la durée du traitement et de la voie d'administration utilisée; elles sont généralement comprises entre 0,05 g et 1 g par jour par voie orale pour un adulte avec des doses unitaires allant de 10 mg à 500 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 cm³
- Benzoate de sodium 80 mg
- Ethanol à 95 % 0,4 cm³
- Hydroxyde de sodium 24 mg
- Propylène glycol 1,6 cm³
- Eau q.s.p. 4 cm³

## Revendications

1. Composés de formule : 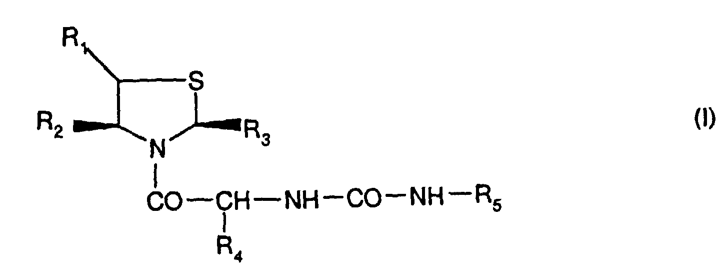 dans laquelle
R₁ représente un radical -(CH₂)ₙ-COORa ou -(CH₂)ₙ-CONRbRc,
R₂ représente un radical phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, trifluorométhyle et trifluorométhoxy,
R₃ représente un radical -COORd ou -CONReRf,
R₄ représente un atome d'hydrogène ou un radical alkyle,
R₅ représente un radical phényle éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk, -C(=NOH)-COOX, -S-alk-COOX, -SO-alk-COOX, -SO₂₋alk-COOX, -O-CH₂-alk'-COOX, -CX=N-O-alk-COOX, -alk-N(OH)-CO-alk, -alk-SO₂H, -SO₂-NH-CO-Rg, -SO₂-NH-SO₂-Rg, -CO-NH-CO-Rg, -CO-NH-SO₂-Rg, -B(OH)₂, -C(NH₂)=NOH, -SO₂-NH-Rh, -CO-NH-Rh, 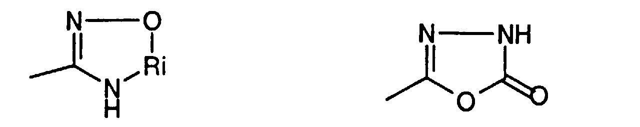 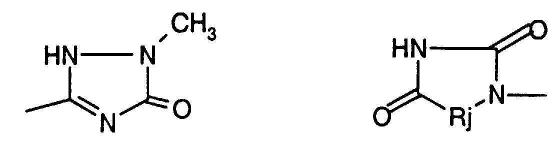 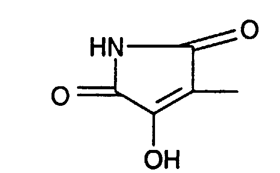 ou diméthyl-2,2 dioxo-4,6 dioxanne-1,3-yl-5,
Ra représente un atome d'hydrogène ou un radical alkyle,
Rb représente un atome d'hydrogène ou un radical alkyle,
Rc représente un radical alkyle, tétrazolyl-5, phénylalkyle dont le noyau phényle est éventuellement substitué par un ou plusieurs substituants choisis parmi les atomes d'halogène et les radicaux alkyle, alcoxy et alkylthio ou phényle éventuellement substitué par un ou plusieurs substituants choisis parmi les atomes d'halogène et les radicaux alkyle, alcoxy et alkylthio,
ou bien Rb et Rc forment avec l'atome d'azote auquel ils sont rattachés un hétérocycle mono ou polycyclique saturé ou insaturé contenant 4 à 9 atomes de carbone et un ou plusieurs hétéroatomes choisis parmi l'oxygène, l'azote ou le soufre et éventuellement substitué par un ou plusieurs radicaux alkyle,
Rd représente un radical alkyle,
Re représente un atome d'hydrogène ou un radical alkyle,
Rf représente un radical alkyle, cycloalkyle ou cycloalkylalkyle,
ou bien Re et Rf forment avec l'atome d'azote auquel ils sont rattachés un hétérocycle mono ou polycyclique saturé ou insaturé contenant 4 à 9 atomes de carbone et un ou plusieurs hétéroatomes choisis parmi l'oxygène, l'azote ou le soufre et éventuellement substitué par un ou plusieurs radicaux alkyle,
Rg représente un radical alkyle, cycloalkyle, trifluorométhyle, phényle éventuellement substitué par un ou plusieurs substituants choisis parmi les radicaux cyano, alcoxy, nitro, amino et les atomes d'halogène,
Rh représente un radical tétrazolyl-5,
Ri représente C=O ou S=O,
Rj représente O ou C=O,
n est égal à 0 ou 1,
X représente un atome d'hydrogène ou un radical alkyle ou phénylalkyle,
alk représente un radical alkyle ou alkylène,
alk' représente un radical hydroxyalkyle, hydroxyalkylène, alcoxyalkyle ou alcoxyalkylène
étant entendu que les radicaux et portions alkyle, alkylène et alcoxy contiennent 1 à 4 atomes de carbone en chaîne droite ou ramifiée et les radicaux ou portions cycloalkyle contiennent 3 à 6 atomes de carbone,
ainsi que leurs isomères et les sels de ces composés.

2. Composés de formule (I) selon la revendication 1 pour lesquels Rb et Rc forment avec l'atome d'azote auquel ils sont rattachés un hétérocycle mono ou polycyclique saturé ou insaturé contenant 4 à 9 atomes de carbone et un ou plusieurs hétéroatomes choisis parmi l'oxygène, l'azote ou le soufre et éventuellement substitué par un ou plusieurs radicaux alkyle celui-ci est de préférence un reste morpholinyle, thiomorpholinyle, pipéridyle éventuellement substitué par un ou plusieurs radicaux alkyle, pyrrolidinyle, tétrahydro-1,2,3,4 quinolyle ou N-alkyl pipérazinyle.

3. Composés de formule (I) selon la revendication 1 pour lesquels Re et Rf forment avec l'atome d'azote auquel ils sont rattachés un hétérocycle mono ou polycyclique saturé ou insaturé contenant 4 à 9 atomes de carbone et un ou plusieurs hétéroatomes choisis parmi l'oxygène, l'azote ou le soufre et éventuellement substitué par un ou plusieurs radicaux alkyle celui-ci est de préférence un reste morpholinyle, thiomorpholinyle, pipéridyle éventuellement substitué par un ou plusieurs radicaux alkyle, pyrrolidinyle, tétrahydro-1,2,3,4 quinolyle ou N-alkyl pipérazinyle.

4. Composés de formule (I) selon la revendication 1 pour lesquels
R₁ représente un radical -(CH₂)ₙ-COORa ou -(CH₂)ₙ-CONRbRc,
R₂ représente un radical phényle éventuellement substitué par un ou plusieurs substituants choisis parmi les atomes d'halogène et les radicaux alkyle, alcoxy, hydroxy, alcoxycarbonyle, trifluorométhyle et trifluorométhoxy,
R₃ représente un radical -COORd ou -CONReRf,
R₄ représente un atome d'hydrogène ou un radical alkyle (1 ou 2C),
R₅ représente un radical phényle éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H,
Ra représente un atome d'hydrogène ou un radical alkyle,
Rb représente un atome d'hydrogène ou un radical alkyle,
Rc représente un radical alkyle, tétrazolyl-5, phénylalkyle dont le noyau phényle est éventuellement substitué par un ou plusieurs substituants choisis parmi les atomes d'halogène et les radicaux alkyle, alcoxy et alkylthio ou phényle éventuellement substitué par un ou plusieurs substituants choisis parmi les atomes d'halogène et les radicaux alkyle, alcoxy et alkylthio,
ou bien Rb et Rc forment avec l'atome d'azote auquel ils sont rattachés un hétérocycle mono ou polycyclique saturé ou insaturé contenant 4 à 9 atomes de carbone et un ou plusieurs hétéroatomes choisis parmi l'oxygène, l'azote ou le soufre et éventuellement substitué par un ou plusieurs radicaux alkyle,
Rd représente un radical alkyle,
Re représente un atome d'hydrogène ou un radical alkyle,
Rf représente un radical alkyle, cycloalkyle ou cycloalkylalkyle,
ou bien Re et Rf forment avec l'atome d'azote auquel ils sont rattachés un hétérocycle mono ou polycyclique saturé ou insaturé contenant 4 à 9 atomes de carbone et un ou plusieurs hétéroatomes choisis parmi l'oxygène, l'azote ou le soufre et éventuellement substitué par un ou plusieurs radicaux alkyle,
n est égal à 0 ou 1,
X représente un atome d'hydrogène ou un radical alkyle ou phénylalkyle,
alk représente un radical alkyle ou alkylène,
alk' représente un radical hydroxyalkyle, hydroxyalkylène, alcoxyalkyle ou alcoxyalkylène,
étant entendu que les radicaux et portions alkyle, alkylène et alcoxy contiennent 1 à 4 atomes de carbone en chaîne droite ou ramifiée et les radicaux ou portions cycloalkyle contiennent 3 à 6 atomes de carbone,
leurs isomères et leurs sels

5. Les composés suivants :
méthoxycarbonyl-5 {[(méthyl-3 phényl)-3 uréido]-2 acétyl}-3 phényl-4 thiazolidinecarboxylate-2 de tert-butyle-(2S,4S,5R),
acide {[(tert-butoxycarbonyl-2 carboxy-5 phényl-4 thiazolidinyl-3-(2S,4S,5R))-2 oxo-2 éthyl]-3 uréido}-3 phénylacétique,
acide {{[(tert-butoxycarbonyl-2 carboxy-5 phényl-4 thiazolidinyl-3-(2S, 4S, 5R))-2 oxo-2 éthyl]-3 uréido}-3 phényl}-2 propionique-(S),
méthoxycarbonyl-5 {[(méthyl-3 phényl)-3 uréido]-2 acétyl}-3 (fluoro-2 phényl)-4 thiazolidinecarboxylate-2 de tert-butyle-(2S,4S,5R),
acide tert-butoxycarbonyl-2 {[(méthyl-3 phényl)-3 uréido]-2 acétyl}-3 (fluoro-2 phényl)-4 thiazolidinecarboxylique-5-(2S,4S,5R),
et leurs sels.

6. Procédé de préparation des composés de formule (I) selon la revendication 1 pour lesquels R₁ représente un radical -(CH₂)ₙ-COORa, n est égal à 0 ou 1, Ra représente un radical alkyle et R₅ représente un radical phényle éventuellement substitué par un ou plusieurs substituants choisis parmi les atomes d'halogène et les radicaux alkyle, alcoxy, alkylthio, trifluorométhyle, alcoxycarbonyle, nitro, acyle, cyano, sulfamoyle, carbamoyle, alcoxyiminoalkyle, alcoxyaminocarbonyle, trifluorométhylsulfonamido, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -S-alk-COOX, -SO-alk-COOX, -SO₂-alk-COOX, -O-CH₂-alk'-COOX, -CX=N-O-alk-COOX dans lesquels X est un radical alkyle ou phénylalkyle caractérisé en ce que l'on fait réagir un dérivé de formule : 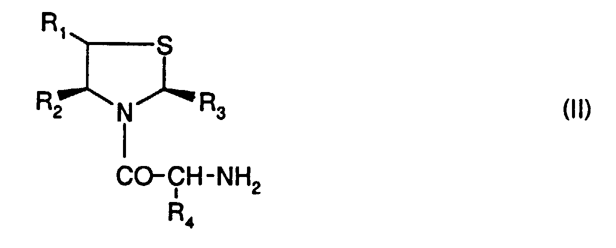 dans laquelle R₁ représente un radical -(CH₂)ₙ-COORa, n est égal à 0 ou 1, Ra représente un radical alkyle, R₂, R₃ et R₄ ont les mêmes significations que dans la revendication 1, sur un phénylisocyanate dont le noyau phényle est éventuellement substitué par un ou plusieurs substituants choisis parmi les atomes d'halogène et les radicaux alkyle, alcoxy, alkylthio, trifluorométhyle, alcoxycarbonyle, nitro, acyle, cyano, sulfamoyle, carbamoyle, alcoxyiminoalkyle, alcoxyaminocarbonyle, trifluorométhylsulfonamido, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -S-alk-COOX, -SO-alk-COOX, -SO₂-alk-COOX, -O-CH₂-alk'-COOX, -CX=N-O-alk-COOX dans lesquels X est un radical alkyle ou phénylalkyle, alk représente un radical alkyle ou alkylène, alk' représente un radical hydroxyalkyle, hydroxyalkylène, alcoxyalkyle ou alcoxyalkylène, isole le produit et le transforme éventuellement en sel.

7. Procédé de préparation des composés de formule (I) selon la revendication 1 pour lesquels R₁ représente un radical -(CH₂)ₙ-COORa, n est égal à 0 ou 1 et Ra est un radical alkyle caractérisé en ce que l'on fait réagir un dérivé réactif de l'acide carbamique, obtenu éventuellement in situ par action d'un dérivé réactif de l'acide carbonique choisi parmi le N,N'-diimidazole carbonyle, le phosgène, le diphosgène, le triphosgène et le chloroformiate de p-nitrophényle sur un dérivé de formule : 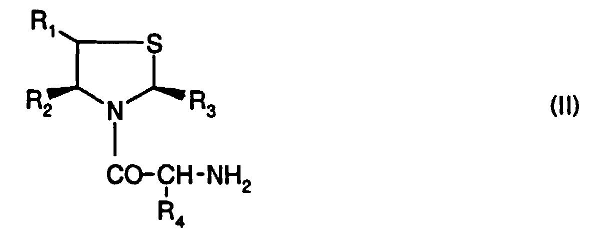 dans laquelle R₁ représente un radical -(CH₂)ₙ-COORa, Ra représente un radical alkyle, R₂, R₃ et R₄ ont les mêmes significations que dans la revendication 1 sur une aniline dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-Rg, -SO₂-NH-SO₂-Rg, -CO-NH-CO-Rg, -CO-NH-SO₂-Rg, -B(OH)₂, -C(NH₂)=NOH, -SO₂-NH-Rₕ, -CO-NH-Rh, 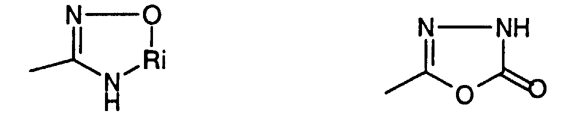 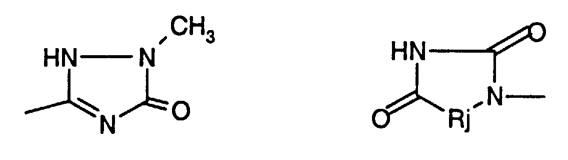 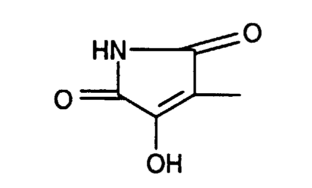 ou diméthyl-2,2 dioxo-4,6 dioxanne-1,3-yl-5, Ri représente C=O ou S=O, Rj représente O ou C=O, n est égal à 0 ou 1, X représente un atome d'hydrogène ou un radical alkyle ou phénylalkyle, alk représente un radical alkyle ou alkylène et alk' représente un radical hydroxyalkyle, hydroxyalkylène, alcoxyalkyle ou alcoxyalkylène, isole le produit et le transforme éventuellement en sel.

8. Procédé de préparation des composés de formule (I) selon la revendication 1 pour lesquels R₁ représente un radical -(CH₂)ₙ-CONRbRC caractérisé en ce que l'on fait réagir un composé de formule (I) correspondant pour lequel R₁ représente un radical -(CH₂)ₙ-COORa, n est égal à 0 ou 1 et Ra représente un atome d'hydrogène, sur une amine de formule HNRbRc dans laquelle Rb et Rc ont les mêmes significations que dans la revendication 1, isole le produit et le transforme éventuellement en sel.

9. Procédé de préparation des composés de formule (I) selon la revendication 1 pour lesquels R₁ représente un radical -(CH₂)ₙ-COORa, n est égal à 0 ou 1 et Ra représente un atome d'hydrogène caractérisé en ce que l'on hydrolyse un composé de formule (I) correspondant pour lequel R₁ représente un radical -(CH₂)ₙ-COORa pour lequel n est égal à 0 ou 1 et Ra représente un radical alkyle, isole le produit et le transforme éventuellement en sel.

10. Médicaments contenant en tant que principe actif au moins un composé de formule (I) selon la revendication 1.

## Patentansprüche

1. Verbindungen der Formel: 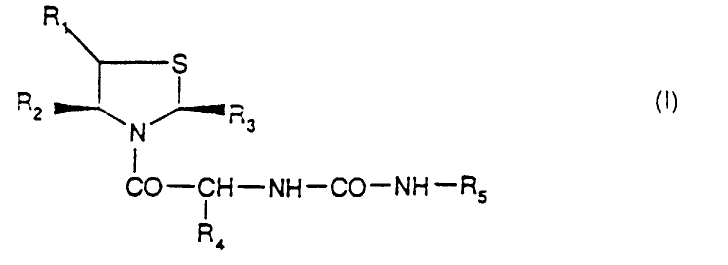 in der
R₁ einen Rest -(CH₂)ₙ-COORa oder -(CH₂)ₙ-CONRbRc darstellt,
R₂ einen Phenylrest darstellt, der gegebenenfalls mit einem oder mehreren Substituenten, die unter den Halogenatomen und den Alkyl-, Alkoxy-, Hydroxy-, Nitro-, Amino-, Monoalkylamino-, Dialkylamino-, Alkoxycarbonyl-, Trifluormethyl- und Trifluormethoxyresten ausgewählt sind, substituiert ist,
R₃ einen Rest -COORd oder -CONReRf darstellt,
R₄ ein Wasserstoffatom oder einen Alkylrest darstellt,
R₅ einen Phenylrest darstellt, der gegebenenfalls mit einem oder mehreren Substituenten, die unter den Halogenatomen und den Alkyl-, Alkoxy-, Alkylthio-, Trifluormethyl-, Carboxy-, Alkoxycarbonyl-, Hydroxy-, Nitro-, Amino-, Acyl-, Cyano-, Sulfamoyl-, Carbamoyl-, Hydroxyiminoalkyl-, Alkoxyiminoalkyl-, Hydroxyaminocarbonyl-, Alkoxyaminocarbonyl-, 5-Tetrazolyl-, 5-Tetrazolyl-alkyl-, Trifluormethylsulfonamido-, Alkylsulfinyl-, Mono- oder Polyhydroxyalkyl-, Sulforesten, den Resten -alk-O-CO-alk, -alk-COOX, -alk-O-alk, -alk'-COOX, -O-alk-COOX, -CH=CH-COOX, -CO-COOX, -alk-SO3H (in Form von Salz), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-Rg, -SO₂-NH-SO₂-Rg, -CO-NH-CO-Rg, -CO-NH-SO₂-Rg, -B(OH)₂, -C(NH₂)=NOH, -SO₂-NH-Rh, -CO-NH-Rh, 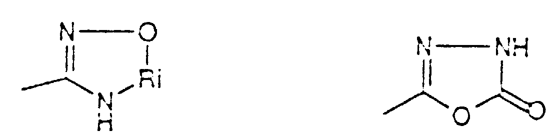 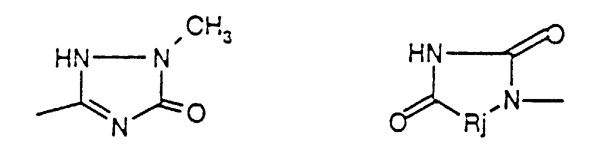 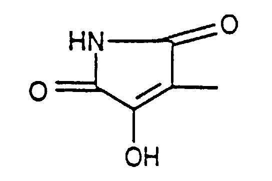
oder dem 2,2-Dimethyl-4,6-dioxo-1,3-dioxan-5-yl-Rest ausgewählt sind, substituiert ist,
Ra ein Wasserstoffatom oder einen Alkylrest darstellt,
Rb ein Wasserstoffatom oder einen Alkylrest darstellt,
Rc einen Alkyl-, 5-Tetrazolyl-, Phenylalkylrest, dessen Phenylkern gegebenenfalls mit einem oder mehreren Substituenten, die unter den Halogenatomen und den Alkyl-, Alkoxy- und Alkylthioresten ausgewählt sind, substituiert ist, oder Phenylrest, der gegebenenfalls mit einem oder mehreren Substituenten, die unter den Halogenatomen und den Alkyl-, Alkoxy- und Alkylthioresten ausgewählt sind, substituiert ist, darstellt,
oder aber Rb und Rc mit dem Stickstoffatom, mit dem sie verbunden sind, einen mono- oder polycyclischen, gesättigten oder ungesättigten Heterocyclus bilden, der 4 bis 9 Kohlenstoffatome und ein oder mehrere unter Sauerstoff, Stickstoff oder Schwefel ausgewählte Heteroatome enthält und gegebenenfalls mit einem oder mehreren Alkylresten substituiert ist,
Rd einen Alkylrest darstellt,
Re ein Wasserstoffatom oder einen Alkylrest darstellt,
Rf einen Alkyl-, Cycloalkyl- oder Cycloalkylalkylrest darstellt,
oder aber Re und Rf mit dem Stickstoffatom, mit dem sie verbunden sind, einen mono- oder polycyclischen, gesättigten oder ungesättigten Heterocyclus bilden, der 4 bis 9 Kohlenstoffatome und ein oder mehrere unter Sauerstoff, Stickstoff oder Schwefel ausgewählte Heteroatome enthält und gegebenenfalls mit einem oder mehreren Alkylresten substituiert ist,
Rg einen Alkyl-, Cycloalkyl-, Trifluormethyl-, Phenylrest, der gegebenenfalls mit einem oder mehreren Substituenten, die unter den Cyano-, Alkoxy-, Nitro-, Aminoresten und den Halogenatomen ausgewählt sind, substituiert ist, darstellt,
Rh einen 5-Tetrazolylrest darsteilt,
Ri C=O oder S=O darstellt,
Rj O oder C=O darstellt,
n gleich 0 oder 1 ist,
X ein Wasserstoffatom oder einen Alkyl- oder Phenylalkylrest darstellt,
alk einen Alkyl- oder Alkylenrest darstellt,
alk' einen Hydroxyalkyl-, Hydroxyalkylen-, Alkoxyalkyl- oder Alkoxyalkylenrest darstellt,
wobei es sich versteht, daß die Alkyl-, Alkylen- und Alkoxyreste und -anteile 1 bis 4 Kohlenstoffatome in gerader oder verzweigter Kette enthalten und die Cycloalkylreste oder -anteile 3 bis 6 Kohlenstoffatome enthalten,
sowie ihre Isomere und die Salze dieser Verbindungen.

2. Verbindungen der Formel (I) gemäß Anspruch 1, für die Rb und Rc mit dem Stickstoffatom, mit dem sie verbunden sind, einen mono- oder polycyclischen, gesättigten oder ungesättigten Heterocyclus bilden, der 4 bis 9 Kohlenstoffatome und ein oder mehrere unter Sauerstoff, Stickstoff oder Schwefel ausgewählte Heteroatome enthält und gegebenenfalls mit einem oder mehreren Alkylresten substituiert ist, dieser ist vorzugsweise ein Morpholinyl-, Thiomorpholinyl-, Piperidylrest, gegebenenfalls substituiert mit einem oder mehreren Alkylresten, Pyrrolidinyl, 1,2,3,4-Tetrahydrochinolyl oder N-Alkyl-piperazinyl.

3. Verbindungen der Formel (I) gemäß Anspruch 1, für die Re und Rf mit dem Stickstoffatom, mit dem sie verbunden sind, einen mono- oder polycyclischen, gesättigten oder ungesättigten Heterocyclus bilden, der 4 bis 9 Kohlenstoffatome und ein oder mehrere unter Sauerstoff, Stickstoff oder Schwefel ausgewählte Heteroatome enthält und gegebenenfalls mit einem oder mehreren Alkylresten substituiert ist, dieser ist vorzugsweise ein Morpholinyl-, Thiomorpholinyl-, Piperidylrest, gegebenenfalls substituiert mit einem oder mehreren Alkylresten, Pyrrolidinyl, 1,2,3,4-Tetrahydrochinolyl oder N-Alkyl-piperazinyl.

4. Verbindungen der Formel (I) gemäß Anspruch 1, für die
R₁ einen Rest -(CH₂)ₙ-COORa oder -(CH₂)ₙ-CONRbRc darstellt,
R₂ einen Phenylrest darstellt, der gegebenenfalls mit einem oder mehreren Substituenten, die unter den Halogenatomen und den Alkyl-, Alkoxy-, Hydroxy-, Alkoxycarbonyl-, Trifluormethyl- und Trifluormethoxyresten ausgewählt sind, substituiert ist,
R₃ einen Rest -COORd oder -CONReRf darstellt,
R₄ ein Wasserstoffatom oder einen Alkylrest (1 oder 2 C) darstellt,
R₅ einen Phenylrest darstellt, der gegebenenfalls mit einem oder mehreren Substituenten, die unter den Halogenatomen und den Alkyl-, Alkoxy-, Alkylthio-, Trifluormethyl-, Carboxy-, Alkoxycarbonyl-, Hydroxy-, Nitro-, Amino-, Acyl-, Cyano-, Sulfamoyl-, Carbamoyl-, Hydroxyiminoalkyl-, Alkoxyiminoalkyl-, Hydroxyamino-carbonyl-, Alkoxyaminocarbonyl-, 5-Tetrazolyl-, 5-Tetrazolyl-alkyl-, Trifluormethyl-sulfonamido-, Alkylsulfinyl-, Mono- oder Polyhydroxyalkyl-, Sulforesten, den Resten -alk-O-CO-alk, -alk-COOX, -alk-O-alk, -alk'-COOX, -O-alk-COOX, -CH=CH-COOX, -CO-COOX, -alk-SO₃H (in Form von Salz), -CH=CH-alk', -C(=NOH)-COOX, -S-alk-COOX, -SO-alk-COOX, -SO₂-alk-COOX, -O-C H₂-alk'-COOX, -CX=N-O-alk-COOX, -alk-N(OH)-CO-alk, -alk-SO₂H ausgewählt sind, substituiert ist,
Ra ein Wasserstoffatom oder einen Alkylrest darstellt,
Rb ein Wasserstoffatom oder einen Alkylrest darstellt,
Rc einen Alkyl-, 5-Tetrazolyl-, Phenylalkylrest, dessen Phenylkern gegebenenfalls mit einem oder mehreren Substituenten, die unter den Halogenatomen und den Alkyl-, Alkoxy- und Alkylthioresten ausgewählt sind, substituiert ist, oder Phenylrest, der gegebenenfalls mit einem oder mehreren Substituenten, die unter den Halogenatomen und den Alkyl-, Alkoxy- und Alkylthioresten ausgewählt sind, substituiert ist, darstellt,
oder aber Rb und Rc mit dem Stickstoffatom, mit dem sie verbunden sind, einen mono- oder polycyclischen, gesättigten oder ungesättigten Heterocyclus bilden, der 4 bis 9 Kohlenstoffatome und ein oder mehrere unter Sauerstoff, Stickstoff oder Schwefel ausgewählte Heteroatome enthält und gegebenenfalls mit einem oder mehreren Alkylresten substituiert ist,
Rd einen Alkylrest darstellt,
Re ein Wasserstoffatom oder einen Alkylrest darstellt,
Rf einen Alkyl-, Cycloalkyl- oder Cycloalkylalkylrest darstellt,
oder aber Re und Rf mit dem Stickstoffatom, mit dem sie verbunden sind, einen mono- oder polycyclischen, gesättigten oder ungesättigten Heterocyclus bilden, der 4 bis 9 Kohlenstoffatome und ein oder mehrere unter Sauerstoff, Stickstoff oder Schwefel ausgewählte Heteroatome enthält und gegebenenfalls mit einem oder mehreren Alkylresten substituiert ist,
n gleich 0 oder 1 ist,
X ein Wasserstoffatom oder einen Alkyl- oder Phenylalkylrest darstellt, alk einen Alkyl- oder Alkylenrest darstellt,
alk' einen Hydroxyalkyl-, Hydroxyalkylen-, Alkoxyalkyl- oder Alkoxyalkylenrest darstellt,
wobei es sich versteht, daß die Alkyl-, Alkylen- und Alkoxyreste und -anteile 1 bis 4 Kohlenstoffatome in gerader oder verzweigter Kette enthalten und die Cycloalkylreste oder -anteile 3 bis 6 Kohlenstoffatome enthalten,
ihre Isomere und ihre Salze.

5. Die folgenden Verbindungen:
(2S,4S, 5R)-tert.-Butyl-5-methoxycarbonyl-3-{2-[3-(3-methyl-phenyl)ureido]acetyl}-4-phenyl-2-thiazolidincarboxylat,
3-{3-[2-((2S,4S, 5R)-2-tert.-Butoxycarbonyl-5-carboxy-4-phenyl-3-thiazolidinyl)-2-oxo-ethyl]ureido}-phenylessigsäure,
(S)-2-{3-{3-[2-((2S,4S,5R)-2-tert.-Butoxycarbonyl-5-carboxy-4-phenyl-3-thiazolidinyl)-2-oxo-ethyl]ureido}phenyl}-propionsäure,
(2S,4S,5R)-tert.-Butyl-5-methoxycarbonyl-3-{2-[3-(3-methyl-phenyl)ureido]acetyl}-4-(2-fluor-phenyl)-2-thiazolidincarboxylat,
(2S,4S,5R )-2-tert. -Butoxycarbonyl-3-{2-[3-(3-methyl-phenyl )ureido]acetyl}-4-(2-fluorphenyl)-5-thiazolidincarbonsäure
und ihre Salze.

6. Verfahren zur Herstellung der Verbindungen der Formel (I) gemäß Anspruch 1, für die R₁ einen Rest -(CH₂)ₙ-COORa darstellt, n gleich 0 oder 1 ist, Ra einen Alkylrest darstellt und R₅ einen Phenylrest darstellt, der gegebenenfalls mit einem oder mehreren Substituenten, die unter den Halogenatomen und den Alkyl-, Alkoxy-, Alkylthio-, Trifluormethyl-, Alkoxycarbonyl-, Nitro-, Acyl-, Cyano-, Sulfamoyl-, Carbamoyl-, Alkoxyiminoalkyl-, Alkoxyaminocarbonyl-, Trifluormethylsulfonamidoresten, den Resten -alk-O-CO-alk, -alk-COOX, -alk-O-alk, -alk'-COOX, -O-alk-COOX, -CH=CH-COOX, -CO-COOX, -alk-SO₃H (in Form von Salz), -CH=CH-alk', -S-alk-COOX, -SO-alk-COOX, -SO₂-alk-COOX, -O-CH₂-alk'-COOX, -CX=N-O-alk-COOX, in denen X ein Alkyl- oder Phenylalkylrest ist, ausgewählt sind, substituiert ist, dadurch gekennzeichnet, daß man ein Derivat der Formel: 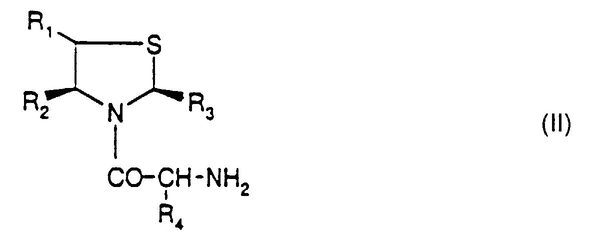 in der R₁ einen Rest -(CH₂)ₙ-COORa darstellt, n gleich 0 oder 1 ist, Ra einen Alkylrest darstellt, R₂, R₃ und R₄ die gleichen Bedeutungen wie in Anspruch 1 haben, mit einem Phenylisocyanat, dessen Phenylkem gegebenenfalls mit einem oder mehreren Substituenten, die unter den Halogenatomen und den Alkyl-, Alkoxy-, Alkylthio-, Trifluormethyl-, Alkoxycarbonyl-, Nitro-, Acyl-, Cyano-, Sulfamoyl-, Carbamoyl-, Alkoxyiminoalkyl-, Alkoxyaminocarbonyl-, Trifluormethylsulfonamidoresten, den Resten -alk-O-CO-alk, -alk-COOX, -alk-O-alk, -alk'-COOX, -O-alk-COOX, -CH=CH-COOX, -CO-COOX, -alk-SO₃H (in Form von Salz), -CH=CH-alk', -S-alk-COOX, -SO-alk-COOX, -SO₂-alk-COOX, -O-CH₂-alk'-COOX, -CX=N-O-alk-COOX, in denen X ein Alkyl- oder Phenylalkylrest ist, alk einen Alkyl-oder Alkylenrest darstellt, alk' einen Hydroxyalkyl-, Hydroxyalkylen-, Alkoxyalkyl-oder Alkoxyalkylenrest darstellt, ausgewählt sind, substituiert ist, umsetzt, das Produkt isoliert und es gegebenenfalls in Salz überführt.

7. Verfahren zur Herstellung der Verbindungen der Formel (I) gemäß Anspruch 1, für die R₁ einen Rest -(CH₂)ₙ-COORa darstellt, n gleich 0 oder 1 ist und Ra ein Alkylrest ist, dadurch gekennzeichnet. daß man ein reaktives Derivat der Carbaminsäure, das gegebenenfalls in situ durch Einwirkung eines reaktiven Derivats der Kohlensäure das unter N,N'-Carbonyl-diimidazol, Phosgen, Diphosgen, Triphosgen und p-Nitrophenyl-Chlorformiat ausgewählt ist, auf ein Derivat der Formel: 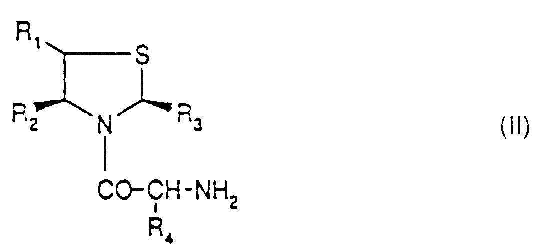 in der R1 einen Rest -(CH₂)ₙ-COORa darstellt, Ra einen Alkylrest darstellt, R₂, R₃ und R₄ die gleichen Bedeuturgen wie in Anspruch 1 haben, erhalten wird, mit einem Anilin, dessen Phenylkern gegebenenfalls mit einem oder mehreren Substituenten, die unter den Halogenatomen und den Alkyl-, Alkoxy-, Alkylthio-, Trifluormethyl-, Carboxy-, Alkoxycarbonyl-, Hydroxy-, Nitro-, Amino-, Acyl-, Cyano-, Sulfamoyl-, Carbamoyl-, Hydroxyiminoalkyl-, Alkoxyiminoalkyl-, Hydroxyaminocarbonyl-, Alkoxyaminocarbonyl-, 5-Tetrazolyl-, 5-Tetrazolyl-alkyl-, Trifluormethylsulfonamido-, Alkylsulfinyl-, Mono- oder Polyhydroxyalkyl-, Sulforesten, den Resten -alk-O-CO-alk, -alk-COOX, -alk-O-alk, -alk'-COOX, -O-alk-COOX, -CH=CH-COOX, -CO-COOX, -alk-SO₃H (in Form von Salz), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-Rg, -SO₂-NH-SO₂-Rg, -CO-NH-CO-Rg, -CO-NH-SO₂-Rg, -B(OH)₂, -C(NH₂)=NOH, -SO₂-NH-Rh, -CO-NH-Rh, 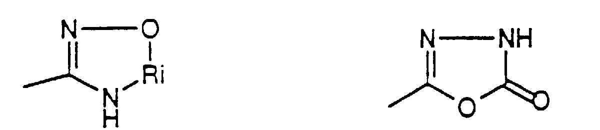 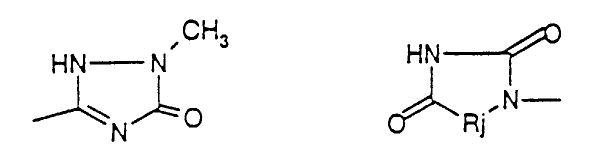 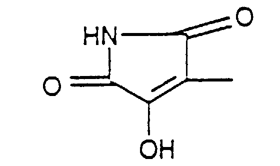 oder dem 2,2-Dimethyl-4,6-dioxo-1,3-dioxan-5-yl-Rest, in denen Ri C=O oder S=O darstellt, Rj O oder C=O darstellt, n gleich 0 oder 1 ist, X ein Wasserstoffatom oder einen Alkyl- oder Phenylalkylrest darstellt, alk einen Alkyl- oder Alkylenrest darstellt und alk' einen Hydroxyalkyl-, Hydroxyalkylen-, Alkoxyalkyl- oder Alkoxyalkylenrest darstellt, ausgewäht sind, substitutert ist, umsetzt, das Produkt isoliert und es gegebenenfalls in Salz überführt.

8. Verfahren zur Herstellung von Verbindungen der Formel (I) gemäß Anspruch 1, für die R₁ einen Rest -(CH₂)ₙ-CONRbRC darstellt, dadurch gekennzeichnet, daß man eine entsprechende Verbindung der Formel (I), für die R₁ einen Rest -(CH₂)ₙ-COORa darstellt, n gleich 0 oder 1 ist und Ra ein Wasserstoffatom darstellt, mit einem Amin der Formel HNRbRc, in der Rb und Rc die gleichen Bedeutungen wie in Anspruch 1 haben, umsetzt, das Produkt isoliert und es gegebenenfalls in Salz überführt.

9. Verfahren zur Herstellung der Verbindungen der Formel (I) gemäß Anspruch 1, für die R₁ einen Rest -(CH₂)ₙ-COORa darstellt, n gleich 0 oder 1 ist und Ra ein Wasserstoffatom darstellt, dadurch gekennzeichnet, daß man eine entsprechende Verbindung der Formel (I), für die R₁ einen Rest -(CH₂)ₙ-COORa darstellt, für den n gleich 0 oder 1 ist und Ra einen Alkylrest darstellt, hydrolysiert, das Produkt isoliert und es gegebenenfalls in Salz überführt.

10. Medikamente, die als Wirkstoff wenigstens eine Verbindung der Formel (I) gemäß Anspruch 1 enthalten.

## Claims

1. Compounds of formula: 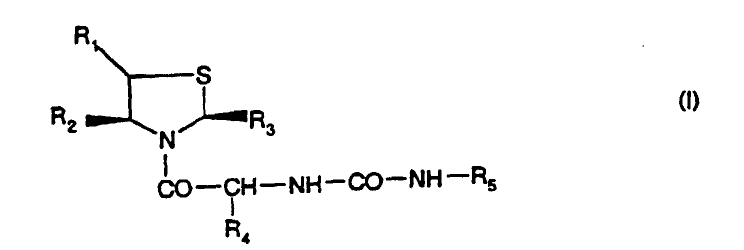 in which
R₁ represents a radical -(CH₂)ₙ-COORa or -(CH₂)ₙ-CONRbRC,
R₂ represents a phenyl radical optionally substituted with one or more substituents chosen from halogen atoms and alkyl, alkoxy, hydroxyl, nitro, amino, monoalkylamino, dialkylamino, alkoxycarbonyl, trifluoromethyl and trifluoromethoxy radicals,
R₃ represents a radical -COORd or -CONReRf,
R₄ represents a hydrogen atom or an alkyl radical,
R₅ represents a phenyl radical optionally substituted with one or more substituents chosen from halogen atoms and alkyl, alkoxy, alkylthio, trifluoromethyl, carboxyl, alkoxycarbonyl, hydroxyl, nitro, amino, acyl, cyano, sulphamoyl, carbamoyl, hydroxyiminoalkyl, alkoxyiminoalkyl, hydroxyaminocarbonyl, alkoxyaminocarbonyl, 5-tetrazolyl, 5-tetrazolylalkyl, trifluoromethylsulphonamido, alkylsulphinyl, mono- or polyhydroxyalkyl, sulpho, -alk-O-CO-alk, -alk-COOX, -alk-O-alk, -alk'-COOX, -O-alk-COOX, -CH=CH-COOX, -CO-COOX, -alk-SO₃H (in salt form), -CH=CH-alk', -C(=NOH)-COOX, -S-alk-COOX, -SO-alk-COOX, -SO₂-alk-COOX, -O-CH₂ -alk' -COOX, -CX=N-O-alk-COOx, -alk-N(OH -CO-alk, -alk-SO₂H, -SO₂-NH-CO-Rg, -SO₂-NH-SO₂-Rg, -CO-NH-CO-Rg, -CO-NH-SO₂-Rg, -B(OH)₂, -C(NH₂)=NOH, -SO₂-NH-Rh, -CO-NH-Rh, 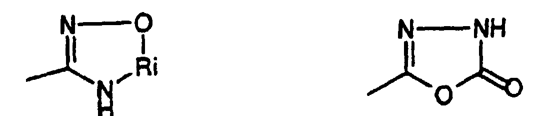 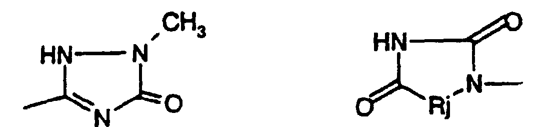 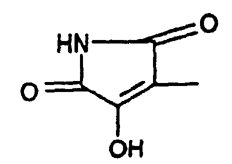 and 2,2-dimethyl-4, 6-dioxo-1,3-dioxan-5-yl radicals, Ra represents a hydrogen atom or an alkyl radical, Rb represents a hydrogen atom or an alkyl radical, Rc represents an alkyl or 5-tetrazolyl radical, a phenylalkyl radical in which the phenyl ring is optionally substituted with one or more substituents chosen from halogen atoms and alkyl, alkoxy and alkylthio radicals or a phenyl radical optionally substituted with one or more substituents chosen from halogen atoms and alkyl, alkoxy and alkylthio radicals,
or alternatively Rb and Rc form, with the nitrogen atom to which they are attached, a saturated or unsaturated mono- or polycyclic heterocycle containing 4 to 9 carbon atoms and one or more hetero atoms chosen from oxygen, nitrogen and sulphur and optionally substituted with one or more alkyl radicals,
Rd represents an alkyl radical,
Re represents a hydrogen atom or an alkyl radical,
Rf represents an alkyl, cycloalkyl or cycloalkylalkyl radical,
or alternatively Re and Rf form, with the nitrogen atom to which they are attached, a saturated or unsaturated mono- or polycyclic heterocycle containing 4 to 9 carbon atoms and one or more hetero atoms chosen from oxygen, nitrogen and sulphur and optionally substituted with one or more alkyl radicals,
Rg represents an alkyl, cycloalkyl or trifluoromethyl radical, or a phenyl radical optionally substituted with one or more substituents chosen from cyano, alkoxy, nitro and amino radicals and halogen atoms,
Rh represents a 5-tetrazolyl radical,
Ri represents C=O or S=O,
Rj represents O or C=O,
n is equal to 0 or 1,
X represents a hydrogen atom or an alkyl or phenylalkyl radical,
alk represents an alkyl or alkylene radical,
alk' represents a hydroxyalkyl, hydroxyalkylene, alkoxyalkyl or alkoxyalkylene radical it being understood that the alkyl, alkylene and alkoxy radicals and portions contain 1 to 4 carbon atoms in a straight or branched chain and the cycloalkyl radicals or portions contain 3 to 6 carbon atoms,
as well as the isomers thereof and the salts of these compounds.

2. Compounds of formula (I) according to claim 1 for which Rb and Rc form, with the nitrogen atom to which they are attached, a saturated or unsaturated mono- or polycyclic heterocycle containing 4 to 9 carbon atoms and one or more hetero atoms chosen from oxygen, nitrogen and sulphur and optionally substituted with one or more alkyl radicals, this heterocycle is preferably a morpholinyl or thiomorpholinyl residue, a piperidyl residue optionally substituted with one or more alkyl radicals, or a pyrrolidinyl, 1,2,3,4-tetrahydroquinolyl or N-alkylpiperazinyl residue.

3. Compounds of formula (I) according to claim 1 for which Re and Rf form, with the nitrogen atom to which they are attached, a saturated or unsaturated mono- or polycyclic heterocycle containing 4 to 9 carbon atoms and one or more hetero atoms chosen from oxygen, nitrogen and sulphur and optionally substituted with one or more alkyl radicals, this heterocycle is preferably a morpholinyl or thiomorpholinyl residue, a piperidyl residue optionally substituted with one or more alkyl radicals, or a pyrrolidinyl, 1,2,3,4-tetrahydroquinolyl or N-alkylpiperazinyl residue.

4. Compounds of formula (I) according to claim 1 for which
R₁ represents a radical -(CH₂)ₙ-COORa or -(CH₂)ₙ-CONRbRc,
R₂ represents a phenyl radical optionally substituted with one or more substituents chosen from halogen atoms and alkyl, alkoxy, hydroxyl, alkoxycarbonyl, trifluoromethyl and trifluoromethoxy radicals,
R₃ represents a radical -COORd or -CONReRf,
R₄ represents a hydrogen atom or an alkyl (1 or 2C) radical,
R₅ represents a phenyl radical optionally substituted with one or more substituents chosen from halogen atoms and alkyl, alkoxy, alkylthio, trifluoromethyl, carboxyl, alkoxycarbonyl, hydroxyl, nitro, amino, acyl, cyano, sulphamoyl, carbamoyl, hydroxyiminoalkyl, alkoxyiminoalkyl, hydroxyaminocarbonyl, alkoxyaminocarbonyl, 5-tetrazolyl, 5-tetrazolylalkyl, trifluoromethylsulphonamido, alkylsulphinyl, mono- or polyhydroxyalkyl, sulpho, -alk-O-CO-alk, -alk-COOX, -alk-O-alk, -alk'-COOK, -O-alk-COOX, -CH=CH-COOX, -CO-COOX, -alk-SO₃H (in salt form), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk and -alk-SO₂H radicals,
Ra represents a hydrogen atom or an alkyl radical, Rb represents a hydrogen atom or an alkyl radical, Rc represents an alkyl or 5-tetrazolyl radical, a phenylalkyl radical in which the phenyl ring is optionally substituted with one or more substituents chosen from halogen atoms and alkyl, alkoxy and alkylthio radicals or a phenyl radical optionally substituted with one or more substituents chosen from halogen atoms and alkyl, alkoxy and alkylthio radicals,
or alternatively Rb and Rc form, with the nitrogen atom to which they are attached, a saturated or unsaturated mono- or polycyclic heterocycle containing 4 to 9 carbon atoms and one or more hetero atoms chosen from oxygen, nitrogen and sulphur and optionally substituted with one or more alkyl radicals,
Rd represents an alkyl radical,
Re represents a hydrogen atom or an alkyl radical, Rf represents an alkyl, cycloalkyl or cycloalkylalkyl radical,
or alternatively Re and Rf form, with the nitrogen atom to which they are attached, a saturated or unsaturated mono- or polycyclic heterocycle containing 4 to 9 carbon atoms and one or more hetero atoms chosen from oxygen, nitrogen and sulphur and optionally substituted with one or more alkyl radicals,
n is equal to 0 or 1,
X represents a hydrogen atom or an alkyl or phenylalkyl radical,
alk represents an alkyl or alkylene radical,
alk' represents a hydroxyalkyl, hydroxyalkylene, alkoxyalkyl or alkoxyalkylene radical,
it being understood that the alkyl, alkylene and alkoxy radicals and portions containing 1 to 4 carbon atoms in a straight or branched chain and the cycloalkyl radicals or portions contain 3 to 6 carbon atoms,
the isomers thereof and the salts thereof

5. The following compounds:
tert-butyl (2S, 4S, 5R)-5-methoxycarbonyl-3-{2-[3-(3-methylphenyl)ureido]acetyl}-4-phenyl-2-thiazolidinecarboxylate,
3-{3-[2-((2S, 4S, 5R)-2-tert-butoxycarbonyl-5-carboxy-4-phenyl-3-thiazolidinyl)-2-oxoethyl]ureido}phenylacetic acid,
(S)-2-{3-{3-[2-((2S, 4S, 5R)-2-tert-butoxycarbonyl-5-carboxy-4-phenyl-3-thiazolidinyl)-2-oxoethyl]ureido}-phenyl}propionic acid,
tert-butyl (2S,4S,5R)-5-methoxycarbonyl-3-{2-[3-(3-methylphenyl)ureido]acetyl}-4-(2-fluorophenyl)-2-thiazolidinecarboxylate,
(2s,45,5R)-2-tert-butoxycarbonyl-3-{2-[3-(3-methylphenyl)ureido]acetyl}-4-(2-fluorophenyl)-5-thiazolidinecarboxylic acid, and the salts thereof.

6. Process for the preparation of the compounds of formula (I) according to claim 1 for which R₁ represents a radical -(CH₂)ₙ-COORa, n is equal to 0 or 1, Ra represents an alkyl radical and R₅ represents a phenyl radical optionally substituted with one or more substituents chosen from halogen atoms and alkyl, alkoxy, alkylthio, trifluoromethyl, alkoxycarbonyl, nitro, acyl, cyano, sulphamoyl, carbamoyl, alkoxyiminoalkyl, alkoxyaminocarbonyl, trifluoromethylsulphonamido, -alk-O-CO-alk, -alk-COOX, -alk-O-alk, -alk'-COOX, -O-alk-COOX, -CH=CH-COOX, -CO-COOX, -alk-SO₃H (in salt form), -CH=CH-alk', -S-alk-COOX, -SO-alk-COOX, -SO₂-alk-COOX, -O-CH₂-alk'-COOX or -CX=N-O-alk-COOX radicals in which X is an alkyl or phenylalkyl radical, characterized in that a derivative of formula: 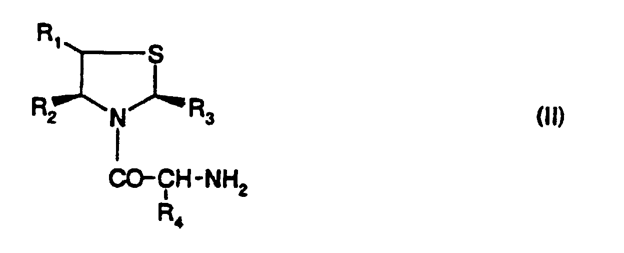 in which R₁ represents a radical -(CH₂)ₙ-COORa, n is equal to 0 or 1, Ra represents an alkyl radical, and R₂, R₃ and R₄ have the same meanings as in claim 1, is reacted with a phenyl isocyanate in which the phenyl ring is optionally substituted with one or more substituents chosen from halogen atoms and alkyl, alkoxy, alkylthio, trifluoromethyl, alkoxycarbonyl, nitro, acyl, cyano, sulphamoyl, carbamoyl, alkoxyiminoalkyl, alkoxyaminocarbonyl, trifluoromethylsulphonamido, -alk-O-CO-alk, -alk-COOX, -alk-O-alk, -alk'-COOX, -O-alk-COOX, -CH=CH-COOX, -CO-COOX, -alk-SO₃H (in salt form), -CH=CH-alk', -S-alk-COOX, -SO-alk-COOX, -SO₂-alk-COOX, -O-CH₂-alk'-COOK or -CX=N-O-alk-COOX radicals in which X is an alkyl or phenylalkyl radical, alk represents an alkyl or alkylene radical, and alk' represents a hydroxyalkyl, hydroxyalkylene, alkoxyalkyl or alkoxyalkylene radical, and the product is isolated and optionally converted into a salt.

7. Process for the preparation of the compounds of formula (I) according to claim 1 for which R₁ represents a radical -(CH₂)ₙ-COORa, n is equal to 0 or 1 and Ra is an alkyl radical, characterized in that a reactive derivative of carbamic acid, optionally obtained in situ by the action of a reactive derivative of carbonic acid chosen from N,N'-carbonyldiimidazole, phosgene, diphosgene, triphosgene and p-nitrophenyl chloroformate on a derivative of formula: 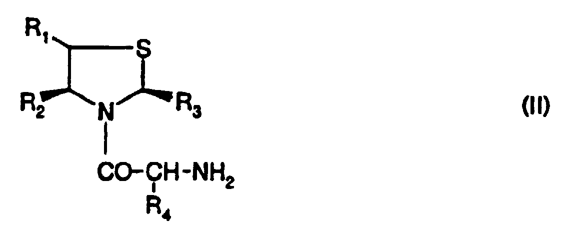 in which R₁ represents a radical -(CH₂)ₙ-COORa, Ra represents an alkyl radical, and R₂, R₃ and R₄ have the same meanings as in claim 1, is reacted with an aniline in which the phenyl ring is optionally substituted with one or more substituents chosen from halogen atoms and alkyl, alkoxy, alkylthio, trifluoromethyl, carboxyl, alkoxycarbonyl, hydroxyl, nitro, amino, acyl, cyano, sulphamoyl, carbamoyl, hydroxyiminoalkyl, alkoxyiminoalkyl, hydroxyaminocarbonyl, alkoxyaminocarbonyl, 5-tetrazolyl, 5-tetrazolylalkyl, trifluoromethylsulphonamido, alkylsulphinyl, mono- or polyhydroxyalkyl, sulpho, -alk-O-CO-alk, -alk-COOX, -alk-O-alk, -alk'-COOX, -O-alk-COOX, -CH=CH-COOX, -CO-COOX, -alk-SO₃H (in salt form), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-Rg, -SO₂-NH-SO₂-Rg, -CO-NH-CO-Rg, -CO-NH-SO₂-Rg, -B(OH)₂, -C(NH₂)=NOH, -SO₂-NH-Rh, -CO-NH-Rh, 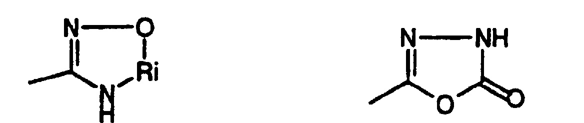 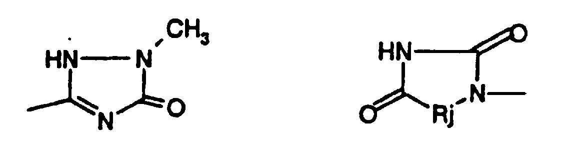 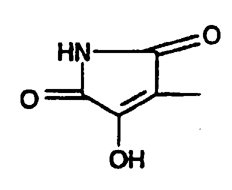 and 2,2-dimethyl-4,6-dioxo-1,3-dioxan-5-yl radicals, Ri represents C=O or S=O, Rj represents O or C=O, n is equal to 0 or 1, X represents a hydrogen atom or an alkyl or phenylalkyl radical, alk represents an alkyl or alkylene radical and alk' represents a hydroxyalkyl, hydroxyalkylene, alkoxyalkyl or alkoxyalkylene radical, and the product is isolated and optionally converted into a salt.

8. Process for the preparation of the compounds of formula (I) according to claim 1 for which R₁ represents a radical -(CH₂)ₙ-CONRbRC, characterized in that a corresponding compound of formula (I), for which R₁ represents a radical -(CH₂)ₙ-COORa, n is equal to 0 or 1 and Ra represents a hydrogen atom, is reacted with an amine of formula HNRbRc in which Rb and Rc have the same meanings as in claim 1, and the product is isolated and optionally converted into a salt.

9. Process for the preparation of the compounds of formula (I) according to claim 1 for which R₁ represents a radical -(CH₂)ₙ-COORa, n is equal to 0 or 1 and Ra represents a hydrogen atom, characterized in that a corresponding compound of formula (I), for which R₁ represents a radical -(CH₂)ₙ-COORa for which n is equal to 0 or 1 and Ra represents an alkyl radical, is hydrolysed, and the product is isolated and optionally converted into a salt.

10. Medicinal products containing at least one compound of formula (I) according to claim 1 as active principle.
